# EUROPÄISCHE PATENTSCHRIFT

(11) **EP 2 863 896 B1**
(45) Veröffentlichungstag und Bekanntmachung des Hinweises auf die Patenterteilung: **14.09.2016**
(21) Anmeldenummer: 13730597.5
(22) Anmeldetag: 24.06.2013
(51) Int. Cl.: A61K 9/16

(54) **ARZNEIFORM ZUR FREISETZUNG VON WIRKSTOFFEN**
DOSAGEFORM FOR THE LIBERATION OF ACTIVE AGENTS
MÉDICAMENT POUR LA LIBÉRATION D'UN AGENT ACTIF

(30) Priorität: 25.06.2012 DE 102012105528
(43) Veröffentlichungstag der Anmeldung: 29.04.2015
(73) Patentinhaber: Hennig Arzneimittel GmbH & Co. KG, 65439 Flörsheim am Main (DE)
(72) Erfinder: FRANCAS, Gernot, 67551 Worms (DE); PRZYKLENK, Karl-Heinz, 64521 Groß-Gerau (DE)
(74) Vertreter: Fuchs Patentanwälte Partnerschaft mbB
(86) Internationale Anmeldenummer: PCT/EP2013/063162
(87) Internationale Veröffentlichungsnummer: WO 2014/001267

(56) Entgegenhaltungen:
- EP-A1- 0 584 594
- WO-A1-2012/110912
- WO-A2-00/35426
- US-A- 4 503 031

## Beschreibung

Die vorliegende Erfindung betrifft eine Arzneiform zur Freisetzung von Wirkstoffen, ein Verfahren zu ihrer Herstellung und ihre Verwendungen.

Diese Erfindung befasst sich mit der Bereitstellung von Arzneiformen, die es ermöglichen, einen Wirkstoff, der üblicherweise zweimal oder mehrmals täglich eingenommen wird, im Wege einer einmal täglichen Gabe wirksam darzureichen.

Bei der Entwicklung neuer Arzneiformen ist es stets von Interesse, die neue Arzneiform so zu gestalten, dass die Patientencompliance erhöht werden kann. Dies gelingt beispielsweise durch Reduktion täglich einzunehmender Einzeldosen eines Arzneimittels. Grund hierfür ist, dass die Patientencompliance zumeist deutlich sinkt, wenn mehrere Einzeldosen eines Arzneimittels am Tag insbesondere noch in Kombination mit weiteren Arzneimitteln einzunehmen sind. Dies zeigt sich umso mehr bei Patienten mit Schluckschwierigkeiten sowie älteren Patienten, die aufgrund von Multimorbidität zahlreiche Arzneimittel einnehmen müssen.

Eine ausreichende Compliance des Patienten ist aber regelmäßig unerlässlich, um Nebenwirkungen und verminderte Wirksamkeit des Arzneimittels zu vermeiden. Die mangelnde Compliance vieler Patienten trägt signifikant zu den steigenden Gesundheitsausgaben bei.

Die Reduktion der Anwendungshäufigkeit eines Arzneimittels von einer mehrmals täglichen auf eine einmal tägliche Gabe hat in dieser Hinsicht an Bedeutung gewonnen. In der Regel soll dabei eine mit mehrmals täglicher Gabe vergleichbare Wirkung erzielt werden. Dies kann mittels Retardierungsmechanismen wie einer Matrixeinbettung eines Arzneistoffs erfolgen, so dass der Wirkstoff verzögert freigesetzt wird, das heißt gleichmäßig über einen längeren Zeitraum.

Die Reduktion der Anwendungshäufigkeit erfordert aber besondere Arzneiformulierungen, an die hohe technologische Anforderungen zu stellen sind, da diese vergleichsweise hohe Wirkstoffmengen aufweisen. Eine technologische Herausforderung besteht darin, eine Arzneiform mit ausreichender Stabilität und gutem Freisetzungsverhalten bei noch tolerierbarer Größe der Arzneiform bereitzustellen. Letzteres ist wichtig, damit die Arzneiform leicht geschluckt werden kann. Ebenso kann es schwierig sein, bei hohen Wirkstoffmengen einen tolerierbaren Geschmack sicherzustellen.

Bislang waren vorwiegend schlecht lösliche Wirkstoffe im Zentrum solcher Entwicklungen. Für diese wurden zahlreiche Arzneiformen entwickelt, insbesondere Matrix-basierte Systeme. Die Resorption solcher Wirkstoffe wird durch ihre Freisetzung aus der Arzneiform bestimmt, so dass durch Entwicklung geeigneter Matrixsysteme eine gute Resorption dieser Wirkstoffe erzielt werden konnte. Für gut lösliche Wirkstoffe werden andere Konzepte benötigt. Naturgemäß ist es wesentlich schwieriger, die Freisetzung eines gut löslichen Wirkstoffes zu verzögern als die eines schlechter löslichen Wirkstoffes, der sich ohnehin langsam auflöst.

Unabhängig von der Löslichkeit eines Wirkstoffs bestehen insbesondere dann erhebliche Schwierigkeiten, wenn Wirkstoffe vorliegen, die aufgrund ihrer Instabilität sehr schwer zu verarbeiten sind. Hierzu zählen insbesondere Wirkstoffe, die sich schnell zersetzen oder hygroskopisch sind.

Hygroskopische Wirkstoffe neigen dazu, Feuchtigkeit aus der Umgebung aufzunehmen, meist in Form von Wasserdampf aus der Luftfeuchtigkeit. Dies kann durch verschiedene Mechanismen wie Absorption und Adsorption erfolgen. Dadurch besteht die Gefahr des Zerfließens oder Verklumpens der Wirkstoffe oder die Bildung unerwünschter Kristallformen. Letzteres erschwert deren Verarbeitung erheblich, so dass aufwendige und kostenintensive Verfahren und Zwischenschritte erforderlich sind, um beispielsweise Feuchtigkeit auszuschließen. Ebenso erschwert diese nachteilige Eigenschaft mancher Wirkstoffe auch die Bereitstellung einer lagerfähigen und haltbaren Arzneiform. Nicht nur die Aufnahme von Feuchtigkeit aus der Umgebung ist kritisch, auch der Arzneiform selbst kann durch den Wirkstoff Feuchtigkeit entzogen werden. Beispielsweise können solche Wirkstoffe Feuchtigkeit aus Kapselhüllen adsorbieren mit der Folge von Rissen in der Kapselhülle.

Durch die Hygroskopizität des Wirkstoffs kann zudem neben der Freisetzung des Wirkstoffs aus der Arzneiform auch die Handhabung solcher Arzneiformen erheblich erschwert sein. Generell wäre es also erforderlich, die Arzneiformen geschützt vor Luft und geringster Feuchtigkeit zu lagern. Allerdings kann die Handhabung beim Patienten nicht kontrolliert werden und es ist davon auszugehen, dass gerade ältere Menschen ihre Tabletten außerhalb des Blisters beispielsweise in Tablettenboxen lagern.

Problematisch ist insofern, dass viele hochwirksame Wirkstoffe und Wirkstoffe, für die keine Alternativen zur Verfügung stehen, hygroskopisch sind. Solche Arzneistoffe finden sich beispielsweise in den Arzneistoffklassen der Antiepileptika, Antibiotika, Antidiabetika, Herz-Kreislauf-Medikamente, Magen-Darm-Therapeutika, Lipidsenker, Antidementiva, Muskelrelaxanzien, Analgetika, Broncholytika/Antiasthmatika oder auch Antiallergika sowie Antiemetika/Antivertiginosa.

Beispielhaft sei hier auf die Antiepileptika Valproinsäure und Carbamazepin verwiesen. Ebenfalls hygroskopische Eigenschaften zeigen die Antibiotika Tetracyclin, Lincomycin, Clindamycin oder auch Rifampicin. Herz-Kreislauf-Medikamente mit hygroskopischen Eigenschaften sind beispielsweise Atenolol und Minoxidil. Als Beispiel für ein Antiemetikum/Antivertiginosum sei Betahistin genannt. Ein Antiallergikum ist beispielsweise Dexamethason, ein Antidementivum ist Piracetam. Ein hygroskopischer Lipidsenker wäre beispielsweise Gemfibrozil.

Auf solche Arzneistoffe kann in der Therapie nicht verzichtet werden. So sind zahlreiche der Wirkstoffe auch als unentbehrliche Arzneimittel von der Weltgesundheitsorganisation eingestuft. Daher besteht ein großer Bedarf an Arzneiformen, die solche Arzneistoffe enthalten und dennoch lagerfähig und haltbar sind sowie ein Freisetzungsverhalten zeigen, das die einmal tägliche Einnahme ermöglicht. Insbesondere besteht ein Bedarf an Arzneiformen, die solche Arzneistoffe so freisetzen, dass eine mehrmals tägliche Einnahme entbehrlich ist, und sich dabei kosteneffektiv und in größerem Maßstab herstellen lassen.

Erhebliche Schwierigkeiten bestehen insbesondere hinsichtlich hygroskopischer Wirkstoffe, die gleichzeitig hoch löslich sind. Die hohe Löslichkeit solcher Wirkstoffe erschwert die ohnehin aufgrund der Hygroskopizität sehr schwierige Bereitstellung solcher Wirkstoffe in Arzneiformen mit zeitlich verzögerter Freisetzung. Aufgrund der hohen Löslichkeit sind übliche Retardformulierungen zur Verzögerung der Freisetzung solcher Wirkstoffe im Allgemeinen ungeeignet. Zusätzliche Schwierigkeiten ergeben sich auch bei Wirkstoffen, die neben der Hygroskopizität eine hohe Azidität aufweisen, insbesondere wenn der Wirkstoff gleichzeitig hoch löslich ist. In diesem Fall besteht zusätzlich die Gefahr, dass der azide Wirkstoff die Hilfsstoffe angreift oder zersetzt. Dies könnte zum Zerfall der Arzneiform und zur Veränderung des Freisetzungsprofils führen. Soll eine Arzneiform mit entsprechender Freisetzung bereitgestellt werden, so besteht also die Gefahr einer schnellen Freisetzung des gesamten Wirkstoffanteils mit resultierenden hohen Plasmaspiegeln und damit starken Nebenwirkungen. Insofern ist die Formulierung eines solchen Arzneistoffs weiter erschwert. Hierbei ist insbesondere Betahistin beispielsweise in Form der Salze Betahistindimesilat oder Betahistindihydrochlorid zu nennen.

Vielfach wurden Arzneiformen bereitgestellt, die jedoch nicht zur Freigabe hygroskopischer Arzneistoffe, insbesondere leicht löslicher und/oder azider Wirkstoffe, geeignet sind, und insofern auch nicht die Probleme bei deren Verarbeitung sowie der Bereitstellung ausreichend stabiler und lagerfähiger Arzneiformen adressieren.

Eine Tablette zur Freisetzung von Betahistin in Form von Betahistindihydrochlorid ist in WO 00/53162 A1 beschrieben. Die Tablette setzt den Wirkstoff dabei verzögert über einen Zeitraum von zumeist 5 bis 8 Stunden frei. Damit ist jedoch eine mehrmals tägliche Gabe nicht entbehrlich. Auch ist auf eine kombinierte schnelle und verzögerte Freisetzung in Form einer Zweischichttablette allgemein verwiesen, ohne dass deren genaue Ausgestaltung oder Herstellung offenbart ist. Insbesondere ist nicht zu entnehmen, wie die Schichten zusammenhalten und wie eine akzeptable Größe der Arzneiform sichergestellt wird. Auch bleibt das Freisetzungsverhalten der Arzneiform unklar. Nur allgemein ist darauf verwiesen, dass eine zweimal oder auch einmal tägliche Gabe möglich ist. Insofern kann nicht entnommen werden, ob die Arzneiform überhaupt geeignet wäre, eine mehrmals tägliche Gabe nachzuahmen.

In DE 100 10 509 A1 sind orale Arzneiformen beschrieben, die einen Saccharosefettsäureester als alleiniges freisetzungssteuerndes Mittel enthalten in einem Anteil von 1 bis 95 Gew.-%. Die Arzneiformen setzen die enthaltenen Wirkstoffe schnell oder retardiert frei. Als mögliche Wirkstoffe ist auf eine Vielzahl an Substanzen verwiesen einschließlich praktisch unlöslicher Wirkstoffe. Beispielsweise ist auch Betahistindimesilat als ein möglicher Wirkstoff genannt. Eine Arzneiform, die geeignet ist, ein und denselben Wirkstoff wenigstens zweiphasig freizusetzen, also eine mehrmals tägliche Einnahme nachzuahmen, ist in DE 100 10 509 A1 nicht beschrieben. Damit ist auch das Problem einer wenigstens zweiphasigen Freisetzung gerade hygroskopischer Wirkstoffe, die zudem unter Umständen noch hoch löslich und azide sind, in DE 100 10 509 A1 nicht gelöst.

WO 98/13029 A1 betrifft wiederum Arzneiformen zur verzögerten Freisetzung von einem oder mehreren Wirkstoffen einschließlich magensaftempfindliche Proteine, Enzyme oder Vakzine, um die therapeutische Wirkung über eine ausreichende Zeit zu gewährleisten. Als ein möglicher Wirkstoff ist unter anderem Betahistin beschrieben. Die Formulierungen enthalten eine wasserunlösliche Beschichtung. Eine magensaftresistente Beschichtung ist nicht beschrieben. Eine schnellfreisetzende Formulierung oder eine andere verzögert freisetzende Formulierung kann mit dieser Formulierung kombiniert werden, beispielsweise in einer Kapsel oder als Beschichtung. Genaue Ausgestaltungen einer solchen Kombination wirkstoffhaltiger Formulierungen, insbesondere geeignete Mengenanteile und - verhältnisse, sind nicht beschrieben. Insofern kann nicht entnommen werden, ob die Arzneiform überhaupt geeignet wäre, eine mehrmals tägliche Gabe optimal nachzuahmen bei sofortiger Freisetzung eines Wirkstoffanteils in einer ersten Phase.

In DE 101 04 880 A1 sind multipartikuläre Arzneiformen zur mehrphasigen Freisetzung einer Vielzahl von Wirkstoffen beschrieben, um eine gleichmäßige Freisetzung der Wirkstoffe über den Darmbereich zu gewährleisten. Als ein möglicher Wirkstoff ist unter anderem auch Betahistin genannt. Die Arzneiform umfasst zwei verschiedene Pelletformen, die in einer Kapsel kombiniert oder zusammen verpresst werden können, wobei ein Verhältnis von 1:1 beschrieben ist. Die verschiedenen Pelletformen setzen den oder die Wirkstoffe jeweils bei unterschiedlichen pH-Werten frei. Die Arzneiform ermöglicht jedoch keine sofortige Freisetzung eines Wirkstoffs in einer ersten Phase. Damit ist es nicht möglich, in einer ersten Phase sehr schnell therapeutische Plasmaspiegel zu erreichen. Die tägliche Einnahme mehrerer Arzneiformen von Wirkstoffen, bei denen schnell therapeutische Plasmaspiegel erreicht werden müssen, wird damit nicht entbehrlich. Außerdem ist die Herstellung der multipartikulären Arzneiformen aufwändig und teuer und insbesondere für hygroskopische und möglicherweise zudem azide Wirkstoffe wenig geeignet.

Insofern besteht ein großer Bedarf an Arzneiformen zur Freisetzung hygroskopischer Wirkstoffe, die mindestens einen Wirkstoff nach Einnahme so freisetzen, dass eine mehrmals tägliche Einnahme entbehrlich ist. Dies betrifft insbesondere Wirkstoffe mit hoher Löslichkeit und/oder hoher Azidität. Herkömmliche Arzneiformen stoßen hier an ihre Grenzen. Insbesondere ist eine Retardierung von Wirkstoffen mit den oben genannten Eigenschaften mit üblichen Retardierungsmitteln und üblicherweise angewandten Retardierungsprinzipien nur mit großem Auswand möglich und sehr kostenintensiv. Eine Verlängerung der Wirkung auf mehr als 10 Stunden ist mit üblichen Retardierungsprinzipien für solche Wirkstoffe kaum möglich. Üblicherweise verwendete Retardierungsmechanismen eignen sich damit nicht zur kosteneffektiven und damit im industriellen Maßstab umsetzbaren Formulierung solcher Wirkstoffe in Arzneiformen, die eine mehrmals tägliche Einnahme des Wirkstoffs entbehrlich machen. Das der vorliegenden Erfindung zugrunde liegende Problem wird durch die Gegenstände der Patentansprüche gelöst.

Die vorliegende Erfindung stellt orale Arzneiformen zur mindestens zweiphasigen Freisetzung mindestens eines Wirkstoffes zur Verfügung, wobei ein Teil des Wirkstoffs in der ersten Phase sofort nach Einnahme in den Magen freigesetzt wird. Außerdem betrifft die Erfindung ein Herstellungsverfahren für solche Arzneiformen sowie geeignete Verwendungen. Das erfindungsgemäße Konzept betrifft also keine verlängerte Freisetzung im Sinne einer herkömmlichen Retardformulierung, sondern sieht die sofortige Freisetzung einer ersten Wirkstoffportion unmittelbar nach der Einnahme und die Freisetzung wenigstens einer zweiten Wirkstoffportion eine gewisse Zeit nach der Einnahme vor. So eine gepulste Freisetzung simuliert die zwei- oder mehrmalige Einnahme eines Wirkstoffes optimal und macht die zwei- oder mehrmals tägliche Einnahme einer Arzneiform entbehrlich.

### Die Arzneiform dieser Erfindung umfasst

- einen Kern
- eine magensaftresistente Zwischenschicht, die auf der Oberfläche des Kerns angeordnet ist, und
- einen Mantel, der auf der dem Kern gegenüberliegenden Seite der Zwischenschicht angeordnet ist,
wobei sowohl der Kern als auch der Mantel jeweils einen Anteil eines Wirkstoffes enthalten. Zusätzlich kann auch die Zwischenschicht wirkstoffhaltig sein. In bevorzugten Ausführungsformen umfasst die erfindungsgemäße Arzneiform keinen zusätzlichen Bestandteil, der wirkstoffhaltig ist, neben dem Kern, der Zwischenschicht und dem Mantel, also insbesondere keine zusätzliche wirkstoffhaltige Schicht. Noch weiter bevorzugt besteht die erfindungsgemäße Arzneiform aus dem Kern, der Zwischenschicht und dem Mantel.

Der Wirkstoff wird aus der Arzneiform mindestens zweiphasig freigesetzt. Weitere Freisetzungsphasen können sich anschließen. Eine "Phase" ist erfindungsgemäß das Zeitintervall einer Freisetzung eines Wirkstoffanteils des Wirkstoffs aus der Arzneiform.

In einer ersten Phase wird ein erster Anteil der Wirkstoffmenge freigesetzt. In einer zweiten Phase wird ein zweiter Anteil der Wirkstoffmenge freigesetzt. Erfindungsgemäß beginnt die erste Phase direkt nach dem Eintritt der Arzneiform in den Magen. Die erste Phase betrifft also die sofortige Freisetzung des Wirkstoffs nach der Einnahme in den Magensaft. Diese erste Phase der Freisetzung erfolgt vorzugsweise aus dem Mantel, wobei der Mantel bevorzugt im Magen zerfällt. Die zweite Phase der Freisetzung erfolgt vorzugsweise aus dem Kern. Der Kern zerfällt vorzugsweise nicht im Magen und wird bevorzugt mit den sogenannten "housekeeper waves", also durch Kontraktionen des Magens, in den Dünndarm transportiert, um dort den Wirkstoffanteil des Kerns freizusetzen.

"Sofortige Freisetzung" bedeutet dabei vorzugsweise, dass mindestens 65% des ersten Anteils der Wirkstoffmenge, bevorzugt mindestens 75% und weiter bevorzugt mindestens 80% des ersten Anteils der Wirkstoffmenge nach 60 Minuten in den Magensaft freigesetzt werden. Besonders bevorzugt werden mehr als 60%, weiter bevorzugt mehr als 65% des ersten Anteils der Wirkstoffmenge bereits nach 30 Minuten in den Magensaft freigesetzt. Dies wird *in-vitro* unter Verwendung gängiger Apparaturen wie einer Drehkörbchen-, Rührblatt- oder Durchflussapparatur in 0,1 N HCl oder Wasser als Prüfmedium unter physiologischen Bedingungen ermittelt (Rotation; bei etwa 36 bis 38°C). Die konkreten Bedingungen hängen vom jeweiligen Wirkstoff und der Arzneiform ab und sind gängigen Arzneibüchern zu entnehmen.

Die Freisetzung des ersten Anteils der Wirkstoffmenge erfolgt also direkt nach der Einnahme der Arzneiform. Bevorzugt wird die Freisetzung dieses Wirkstoffanteils dabei nicht durch Überzüge verzögert. Vor Freisetzung des ersten Wirkstoffanteils muss sich also bevorzugt nicht erst ein etwaiger Überzug, also beispielsweise ein Film, lösen, aufreißen oder quellen.

Bevorzugt beginnt die zweite Phase erst frühestens 4 Stunden, weiter bevorzugt erst frühestens 6 Stunden und am meisten bevorzugt erst frühestens 7 Stunden nach Einnahme der Arzneiform. Eine zu frühe zweite Phase könnte zu erhöhten Plasmakonzentrationen mit entsprechender Gefahr von Nebenwirkungen führen und birgt ferner die Gefahr, dass die Wirkstofffreisetzung nicht ausreichend lange aufrechterhalten wird, damit die einmal täglich Einnahme therapeutisch ausreichend ist. Die Freisetzung des zweiten Wirkstoffanteils erfolgt jedoch vorzugsweise spätestens 16 Stunden nach Einnahme, weiter bevorzugt spätestens 14 Stunden nach Einnahme und am meisten bevorzugt spätestens 12,5 Stunden nach Einnahme. Eine zu späte Freisetzung würde dazu führen, dass der zweite Wirkstoffanteil erst in zu tiefen Darmabschnitten freigesetzt wird, so dass wegen der verminderten Resorptionsfläche und begrenzten Flüssigkeitsmenge die Resorption deutlich vermindert wäre. Besonders bevorzugt erfolgt die Freisetzung des zweiten Wirkstoffanteils im Colon.

Die Freisetzung in der zweiten Phase erfolgt dabei bevorzugt sofort nach dem Erreichen eines bestimmten pH-Bereichs. Der zweite Wirkstoffanteil wird vorzugsweise aus dem Kern der Arzneiform freigesetzt. Es werden also nach Erreichen eines bestimmten pH-Wertes innerhalb von 60 Minuten vorzugsweise mindestens 65% des zweiten Anteils der Wirkstoffmenge, bevorzugt mindestens 75% und weiter bevorzugt mindestens 80% des zweiten Anteils der Wirkstoffmenge freigesetzt, ermittelt *in-vitro* unter Verwendung gängiger Apparaturen in einem Puffermedium mit dem jeweiligen pH-Wert als Prüfmedium unter physiologischen Bedingungen (Rotation; bei etwa 36 bis 38°C). Die konkreten Bedingungen hängen vom jeweiligen Wirkstoff und der Arzneiform ab und sind gängigen Arzneibüchern zu entnehmen.

Alternativ kann die Freisetzung des zweiten Wirkstoffanteils aber auch verzögert erfolgen, das heißt verlängert über ein bestimmtes Zeitintervall, bevorzugt über mindestens 4 Stunden, weiter bevorzugt über mindestens 6 Stunden. In einer Ausführungsform können weitere Freisetzungsphasen vorgesehen sein, in der Anteile des Wirkstoffs freigesetzt werden.

Die Arzneiform kann aber neben dem ersten Wirkstoff noch weitere Wirkstoffe umfassen, die entweder einphasig oder mehrphasig freigesetzt werden. Bevorzugt ist eine mehrphasige Freisetzung. Besonders bevorzugt enthält die erfindungsgemäße Arzneiform nur den einen Wirkstoff. Dadurch können etwaige Inkompatibilitäten bei Herstellung und Lagerung vermieden werden.

Die Arzneiform der vorliegenden Erfindung ist eine orale Arzneiform, das bedeutet, dass sie zur oralen Einnahme bestimmt ist. Erfindungsgemäß kann es sich dabei beispielweise um eine Tablette, Caplette, Mikrotablette, ein Granulat oder Pellets handeln. Die Arzneiform kann auch in einer multipartikulären Arzneiform wie einer Tablette oder Kapsel oder in einem Granulat, Sachet, Stick oder Beutel enthalten sein. Bevorzugt handelt es sich bei der erfindungsgemäßen Arzneiform um eine Tablette.

Ganz besonders bevorzugt handelt es sich bei der erfindungsgemäßen Arzneiform um eine Mantel-Kern-Tablette, die im Inneren den Kern enthält, der von der Zwischenschicht umgeben ist. Diese wiederrum ist von dem Mantel umgeben. "Umgeben" ist der Kern von der Zwischenschicht vorzugsweise dann, wenn mindestens 95% der Gesamtoberfläche des Kerns, weiter bevorzugt mindestens 98% und ganz besonders bevorzugt mindestens 99% der Gesamtoberfläche des Kerns von der Zwischenschicht bedeckt sind. Noch mehr bevorzugt ist die Gesamtoberfläche des Kerns "vollständig" von der Zwischenschicht umgeben, wobei "vollständig" vorzugsweise bedeutet, dass mehr als 99,5%, idealerweise mehr als 99,8% der Gesamtoberfläche des Kerns von der Zwischenschicht bedeckt sind. Dies schließt nicht aus, dass Poren in der Zwischenschicht enthalten sind. "Umgeben" ist die Zwischenschicht vom Mantel vorzugsweise dann, wenn mindestens 95% der Gesamtoberfläche des der dem Kern gegenüberliegenden Seite der Zwischenschicht, weiter bevorzugt mindestens 98% und ganz besonders bevorzugt mindestens 99% von dem Mantel bedeckt sind. Noch mehr bevorzugt ist die Gesamtoberfläche der dem Kern gegenüberliegenden Seite der Zwischenschicht vollständig von dem Mantel umgeben, also sind vorzugsweise mehr als 99,5% und idealerweise mehr als 99,8% der dem Kern gegenüberliegenden Seite der Zwischenschicht vom Mantel bedeckt.

Alternativ kann die Arzneiform jedoch auch als Schichttablette vorliegen, bei der die Zwischenschicht zwischen den Schichten der Schichttablette vorliegen kann, dabei würde der Kern eine der Schichten und der Mantel eine andere Schicht darstellen.

Als besonders vorteilhaft hat sich die Ausgestaltung als Mantel-Kern-Tablette erwiesen. Mit der Ausgestaltung als Mantel-Kern-Tablette konnte eine optimale Initiierung und eine optimale Aufrechterhaltung der Freisetzung des Wirkstoffs über einen ausreichenden Zeitraum erzielt werden. Mit diesem Mantel-Kern-Konzept wird die tägliche Einnahme wenigstens zweier Tabletten vorzugsweise im Abstand von 8 bis 12 Stunden besonders vorteilhaft nachgeahmt.

Der Zweck einer solchen Ausgestaltung der Arzneiform als Mantel-Kern-Tablette weicht von dem im Stand der Technik üblicherweise verfolgten Zweck ab. So sind Mantel-Kern-Tabletten als Formulierungsoption für unverträgliche Wirkstoffe oder extrem schlecht schmeckende oder extrem instabile Wirkstoffe bekannt.

Der Wirkstoff ist vorzugsweise hygroskopisch und gut wasserlöslich. Hygroskopische und gut wasserlösliche Wirkstoffe profitieren besonders vom erfindungsgemäßen Wirkstoffdesign. Ein hygroskopischer Wirkstoff liegt erfindungsgemäß vor, wenn dieser oder dessen Salz dazu neigt, Wasser aus der Umgebung aufzunehmen sei es durch Adsorption oder Absorption oder sonstige Mechanismen. Ebenso erfassen hygroskopische Wirkstoffe damit solche, die durch Wasseraufnahme zerfließen (Deliqueszenz).

Hygroskopizität liegt erfindungsgemäß vorzugsweise dann vor, wenn ein Stoff, der bei 75% relativer Luftfeuchte, bevorzugt bei 45% relativer Luftfeuchte und am meisten bevorzugt bereits bei 30% relativer Luftfeuchte mehr als 1 Gew.-% Wasser aus der Umgebung aufnimmt. Besonders bevorzugt werden mindestens 5 Gew.-%, weiter bevorzugt mindestens 8 Gew.-% Wasser aus der Umgebung aufgenommen. Die Bestimmung der Wasseraufnahme erfolgt durch Einbringen von 0,5 g einer zuvor ausschließlich luftdicht gelagerten Probe eines Stoffes in der Atmosphäre einer relativen Luftfeuchtigkeit von 75%, 45% oder 30% für 24 Stunden. Anschließend wird der Wassergehalt des Stoffes mit der Karl-Fischer-Methode in einem Karl-Fischer-Titrator unter Aufheizen bis auf höchstens 300°C ermittelt. Der Wassergehalt wird mit dem Wassergehalt von 0,5 g des Stoffes, die derselben Probe entstammen, aber weiterhin luftdicht verschlossen gelagert wurden, verglichen.

Bevorzugt wird ein Wirkstoff verwendet, der bei Trocknung einer unter den oben genannten Bedingungen gelagerten Probe bei 105°C bis zur Gewichtskonstanz in einem Trockenschrank oder mittels eines Infrarot-Trockners, bevorzugt ist der Infrarot-Trockner, einen Wassergehalt von mindestens 0,5 Gew.-% bezogen auf die Gesamtmasse der Arzneiform führt, weiter bevorzugt von mindestens 1 Gew.-% aufweist. Durch dieses Verfahren wird der nicht chemisch gebundene Wasseranteil der Probe erfasst. Dabei ist die Probe bevorzugt eine Arzneiform umfassend den Wirkstoff und Hilfsstoffe.

Beispiele bevorzugter Wirkstoffe, die selbst oder in Form ihrer pharmazeutisch annehmbaren Salze hygroskopisch sind, sind Valproinsäure, Carbamazepin, Tetracyclin, Lincomycin, Clindamycin, Rifampicin, Erythromycin, Metformin, Atenolol, Ranitidin, Minoxidil, Acetylsalicylsäure, Diclofenac, Omeprazol, Methyldopa, Betahistin, Dexamethason, Prednisolon, Piracetam, Pravastatin und Gemfibrozil.

Der Wirkstoff ist vorzugsweise gut löslich. Eine gute oder hohe Löslichkeit haben Wirkstoffe erfindungsgemäß dann, wenn 1 g Wirkstoff in maximal 10 ml Wasser bei 15 bis 25°C unter Normaldruck gelöst werden kann.

Besonders bevorzugt ist der Wirkstoff ein Wirkstoff der BCS Klasse I nach dem Biopharmazeutischen Klassifizierungssystem. Der BCS Klasse I gehören solche Wirkstoffe an, die eine hohe Löslichkeit und ein hohes Permeationsvermögen aufweisen ermittelt beispielsweise gemäß den Vorgaben der FDA ("Waiver of In Vivo Bioavailability and Bioequivalence Studies for Immediate-Release Solid Oral Dosage Forms Based on a Biopharmaceutics Classification System", 2000).

Der Wirkstoff kann auch azide sein. Azide ist ein Wirkstoff erfindungsgemäß vorzugsweise dann, wenn dessen wässrige Lösung bei einem Masseanteil des Wirkstoffes von 10 Gew.-% bei Normbedingungen (DIN 1343) einen pH-Wert von höchstens 5 aufweist, weiter bevorzugt einen pH-Wert von höchstens 4 und ganz besonders bevorzugt einen pH-Wert von höchstens 3.

Die erfindungsgemäße Arzneiform umfasst insgesamt vorzugsweise zwischen 1 mg und 200 mg an Wirkstoff. Bevorzugt umfasst die erfindungsgemäße Arzneiform den Wirkstoff in einer Menge zwischen 1,5 mg und 150 mg, weiter bevorzugt in einer Menge zwischen 2 mg und 120 mg, noch mehr bevorzugt in einer Menge zwischen 5 mg und 60 mg.

Vorzugsweise ist der Wirkstoff Betahistin oder ein pharmazeutisch geeignetes Salz von Betahistin. Pharmazeutisch geeignete Salze von Betahistin sind insbesondere Betahistindihydrochlorid, Betahistindimesilat, BetahistinMethansulfonat, Betahistinfumarat und Betahistincitrat. Weiter bevorzugt sind Betahistindihydrochlorid, Betahistindimesilat, Betahistinfumarat und Betahistincitrat. Ganz besonders bevorzugt sind Betahistindihydrochlorid und Betahistindimesilat. In einer besonders bevorzugten Ausführungsform umfasst die Arzneiform Betahistindihydrochlorid oder Betahistindimesilat. Dabei sind bevorzugt zwischen 2 mg und 55 mg bezogen auf die Wirkstoffbase in der Arzneiform enthalten. Die Wirkstoffbase ist Betahistin, also nicht das Salz.

Ganz besonders bevorzugt enthält die erfindungsgemäße Arzneiform Betahistindihydrochlorid, bevorzugt in einer Menge zwischen 8 mg und 100 mg, ganz besonders bevorzugt zwischen 30 und 60 mg, und am meisten bevorzugt sind 48 mg in der Arzneiform enthalten. Wenn in der Anmeldung absolute Werte genannt werden, erfasst dies stets auch Werte im Toleranzbereich von +/- 2% um den genannten absoluten Wert.

Der Wirkstoff kann dabei im Kern, in der Zwischenschicht und im Mantel enthalten sein. Dabei ist es bevorzugt, dass der Wirkstoffanteil im Kern und im Mantel jeweils 40% bis 60% der Gesamtmenge des Wirkstoffes in der Arzneiform beträgt. Der Anteil des Wirkstoffs im Kern beträgt vorzugsweise wenigstens 42% und höchstens 58%, weiter bevorzugt wenigstens 45% und höchstens 55% der Gesamtmenge des Wirkstoffs in der Arzneiform. Sind die Wirkstoffmengen im Kern zu klein, so besteht die Gefahr, dass die Wirkung nicht ausreichend lange aufrechterhalten werden kann.

Vorzugsweise ist der Wirkstoffanteil in Kern und Mantel gleich hoch. Dies ermöglicht eine mehrphasige Freisetzung gleicher Wirkstoffmengen und ist vorteilhaft, weil diese Art der Freisetzung eine mehrmalige Gabe am Tag optimal nachahmt. Alternativ kann die Verteilung des Wirkstoffs im Kern und Mantel auch abweichen. "Gleich hoch" bedeutet erfindungsgemäß bevorzugt, dass das Masseverhältnis der Wirkstoffmenge im Kern zu der Wirkstoffmenge im Mantel vorzugsweise wenigstens 1 zu 1,1 und höchstens 1,1 zu 1, weiter bevorzugt wenigstens 1 zu 1,05 und höchstens 1,05 zu 1 beträgt.

Neben dem Wirkstoff kann ein zweiter, gegebenenfalls auch ein dritter Wirkstoff in Kern, Mantel und/oder Zwischenschicht enthalten sein. Ganz besonders bevorzugt enthält die Zwischenschicht keinen Wirkstoff.

Der Kern ist wirkstoffhaltig, umfasst also mindestens den Wirkstoff. Neben dem Wirkstoff ist bevorzugt mindestens ein pharmazeutisch annehmbarer Hilfsstoff enthalten. Pharmazeutisch annehmbare Hilfsstoffe sind solche, die hinsichtlich der Patientensicherheit sowie Verarbeitbarkeit generell für die Verwendung in Arzneiformen geeignet sind.

Es hat sich als vorteilhaft erwiesen, wenn der Kern mindestens einen Trägerstoff umfasst, der bevorzugt sorptionsaktiv ist. Ein sorptionsaktiver Trägerstoff ist in der Lage, an einen hygroskopischen Wirkstoffanteil angelagert zu werden, also diesen zu "tragen" und die Hygroskopizität des Wirkstoffs zu einem gewissen Grad zu beseitigen. Dadurch wird ein Zerfließen des hygroskopischen Wirkstoffs verhindert, was einen deutlich positiven Einfluss auf die Bruchfestigkeit und Haltbarkeit der erzielten Arzneiform hat.

Überraschenderweise haben sich dabei insbesondere Trägerstoffe als geeignet erwiesen, die selbst hygroskopisch sind, also zur Feuchtigkeitsaufnahme neigen. Eigentlich wäre eine schlechte Verarbeitbarkeit durch die zusätzliche Feuchtigkeitsaufnahme solcher Trägerstoffe zu erwarten. Tatsächlich kann aber durch Verwendung solcher Trägerstoffe zusammen mit dem Wirkstoff ein fester und stabiler Kern erhalten werden, der einfach verarbeitbar und verpressbar ist.

Allerdings hat sich gezeigt, dass der Trägerstoff oder die Trägerstoffe hierfür vorzugsweise in einem Überschuss gegenüber dem Wirkstoff vorhanden sein müssen. Das Masseverhältnis des Trägerstoffs oder der Trägerstoffe im Kern zum Wirkstoff im Kern beträgt daher vorzugsweise mindestens 2:1, weiter bevorzugt mindestens 3:1, weiter bevorzugt mindestens 3,4:1 und in ganz besonders bevorzugten Ausführungsformen mindestens 5:1. Der Trägerstoff hat dabei offenbar die Wirkung, dass eine weitere Wasseraufnahme aus der Umgebung deutlich vermindert werden kann. Das Masseverhältnis sollte aber vorzugsweise nicht höher sein als 20:1, bevorzugt nicht höher als 10:1, weiter bevorzugt nicht höher als 7,5:1, da sonst die Verarbeitbarkeit erschwert sein kann. Auch ist ein zu hohes Masseverhältnis mit einem zu großen Kern verbunden. Dies kann zu einer zu starken Vergrößerung der erfindungsgemäßen Arzneiform führen.

Geeignete Trägerstoffe können anorganischer oder organischer Art sein. Bevorzugte Trägerstoffe sind ausgewählt aus natürlichen und synthetischen Polymeren. Bevorzugt handelt es sich dabei um natürliche oder modifizierte Polysaccharide, vorzugsweise bestehend aus zwei oder mehreren gleichen oder verschiedenen Monosaccharidbausteinen. Solche Stoffe sind kostengünstig erhältlich und leicht verarbeitbar. Letzteres ist insbesondere dann vorteilhaft, wenn die Freisetzung des Wirkstoffanteils aus dem Kern sofort erfolgen soll. Als besonders vorteilhaft haben sich Polysaccharide erwiesen, die Glucoseeinheiten enthalten. Dabei ist das natürliche oder modifizierte Polysaccharid bevorzugt ein Disaccharid, besonders bevorzugt ist dieses ausgewählt aus Lactose, Saccharose und Mischungen davon.

Alternativ kann es sich bei dem Trägerstoff um ein Polysaccharid mit mehr als 10 Monosaccharidbausteinen handeln, das vorzugweise bei Kontakt mit Wasser quellfähig ist. Besonders bevorzugt handelt es sich dabei um Stärke einschließlich von Stärkederivaten, Cellulose einschließlich von Cellulosederivaten oder Mischungen davon. Ganz besonders bevorzugt ist pulverisierte Cellulose wie mikrokristalline Cellulose. Ebenso kann Stärke eingesetzt werden in nativer oder vorverkleisterter Form, insbesondere Maisstärke. Solche Trägerstoffe sind noch besser in der Lage, Wasser von dem Wirkstoffanteil aufzunehmen.

Allerdings zeigte sich, dass die Bruchfestigkeit der Tablette bei ausschließlicher Verwendung solcher Polysaccharide als Trägerstoffe bei Verwendung bestimmter Wirkstoffe mit zunehmender Lagerdauer sinken kann. In einer besonders bevorzugten Ausführungsform sind daher mindestens zwei Trägerstoffe im Kern enthalten, ganz besonders bevorzugt handelt es sich dabei um mindestens ein Disaccharid und um mindestens ein Polysaccharid mit mehr als 10 Monosaccharidbausteinen, das vorzugsweise bei Kontakt mit Wasser quellfähig ist. Durch diese Kombination wurde eine optimale Bruchfestigkeit und Stabilität erzielt. Auch ist der Wirkstoffanteil vorzugsweise auf dem Trägerstoffgemisch optimal fixiert. Ganz besonders bevorzugt enthält der Kern mindestens drei Trägerstoffe. Das Masseverhältnis von Polysacchariden mit mehr als 10 Monosaccharidbausteinen zu Disacchariden beträgt vorzugsweise mindestens 1,1:1, bevorzugt mindestens 1,25:1 und ganz bevorzugt mindestens 1,3:1.

Es können auch anorganische Trägerstoffe, vorzugsweise mit hygroskopischen Eigenschaften eingesetzt werden. Anorganische Trägerstoffe können auch zusätzlich zu den oben genannten organischen Trägerstoffen enthalten sein.

Bevorzugt umfasst der Kern Salze der oben genannten organischen oder anorganischen Substanzen in einem Anteil von maximal 5 Gew.-%, bevorzugt maximal 2 Gew.-% und ganz besonders bevorzugt in einem Anteil von maximal 1 Gew.-%. Werden Salze der oben genannten organischen Substanzen oder Salze anorganischer Substanzen im erfindungsgemäßen Kern in hohen Mengen eingesetzt, kann es zur Reaktion des Wirkstoffs im Kern mit diesen Substanzen kommen, wodurch die Stabilität des Wirkstoffs im Kern nachteilig beeinflusst werden kann. Salze umfassen dabei insbesondere Alkali- und Erdalkalisalze solcher Substanzen. Besonders bevorzugt ist der Kern frei von Salzen der oben genannten organischen oder anorganischen Substanzen, d.h. Salze solcher Substanzen, insbesondere umfassend Calciumphosphat, Natriumsalze von Cellulosen und/oder Magnesiumstearat, sind lediglich als Verunreinigungen in einem Anteil von vorzugsweise maximal 0,5 Gew.-%, bevorzugt von maximal 0,1 Gew.-% an der Gesamtmasse des Kerns enthalten.

Bevorzugt beträgt der Gesamtanteil von erfindungsgemäßen Trägerstoffen an der Gesamtmasse des Kerns mindestens 50 Gew.-%, bevorzugt mindestens 63 Gew.-% und ganz besonders bevorzugt mindestens 65 Gew.-% sowie weiter bevorzugt mindestens 68 Gew.-%. Wird zu wenig an dem Trägerstoff(en) eingesetzt, so kann die Hygroskopizität des Wirkstoffs nur unzureichend kompensiert werden. Außerdem hat der Trägerstoff/haben die Trägerstoffe eine gewisse retardierende Wirkung auf die Freisetzung des Wirkstoffs. Bevorzugt enthält der Kern maximal 90 Gew.-% Trägerstoff bzw. Trägerstoffe, bevorzugt maximal 85 Gew.-% und weiter bevorzugt maximal 80 Gew.-%. Zu hohe Masseanteile von Trägerstoff(en) erschweren die Formgebung des Kerns. Der Trägerstoff kann auch die Azidität des Wirkstoffes abschwächen. Hierfür ist schon eine gewisse Trägerstoffmenge erforderlich.

Damit die Menge des Trägerstoffes aber in einem sinnvollen Rahmen bleibt, kann erfindungsgemäß bevorzugt eine Puffersubstanz im Kern eingesetzt werden. Die eingesetzte Masse an Puffersubstanz ist bevorzugt kleiner als die Masse der Trägerstoffe. Dabei ist ein Masseverhältnis von Trägerstoffen im Kern zu Puffersubstanz im Kern von vorzugsweise mindestens 3:1, weiter bevorzugt mindestens 4:1 und ganz besonders bevorzugt mindestens 4,5:1 vorteilhaft und es konnte sowohl eine ausreichende Kompensation der Hygroskopizität wie auch eine ausreichende Abschwächung der Azidität erreicht werden bei guter Verarbeitbarkeit des Kerns. Der Kern enthält vorzugsweise mindestens 1 Gew.-% Puffersubstanz, bevorzugt mindestens 8 Gew.-%. Vorzugsweise enthält der Kern maximal 26 Gew.-% Puffersubstanz, weiter bevorzugt maximal 16 Gew.-%. Der Anteil an Puffersubstanz sollte bestimmte Werte nicht überschreiten, weil diese Komponente die Stabilität der Arzneiform beeinträchtigen kann.

Als Puffersubstanz sind organische Säuren besonders gut geeignet, vorzugsweise sind diese niedermolekular. Als "niedermolekular" sind organische Säuren zu bezeichnen, die Molmassen unter 300 g/mol aufweisen. Die Puffersubstanz ist bevorzugt eine Carbonsäure ausgewählt aus Citronensäure, Milchsäure, Weinsäure, Fumarsäure, Maleinsäure und Ascorbinsäure sowie Mischungen daraus. Besonders bevorzugt sind alpha-Hydroxycarbonsäuren, die aufgrund der alpha-Hydroxygruppe eine optimale Pufferwirkung zeigen. Bevorzugt sind diese ausgewählt aus Milchsäure, Weinsäure, Citronensäure und Mischungen davon. Ganz besonders vorteilhaft ist Citronensäure, die sich besonders leicht verarbeiten lässt und die Freisetzung des Wirkstoffs aus dem Kern dabei nicht signifikant verändert. Dies ist überraschend, da Citronensäure in vielen Darreichungsformen eine Freisetzungsbeschleunigung bewirkt, was hier unerwünscht wäre.

Das Masseverhältnis des Wirkstoffs im Kern zur Puffersubstanz im Kern liegt vorzugsweise bei mindestens 0,5:1, bevorzugt mindestens 0,8:1, weiter bevorzugt mindestens 0,9:1. Das Masseverhältnis beträgt vorzugweise maximal 1,5:1, weiter bevorzugt maximal 1,2:1 und besonders bevorzugt maximal 1,1:1. Bei zu geringer Menge an Puffersubstanz kann die Azidität des Wirkstoffs nicht ausreichend gepuffert werden. Bei zu hoher Menge an Puffersubstanz nimmt die Azidität wiederrum unvorteilhaft zu. Der Kern enthält erfindungsgemäß bevorzugt mindestens 8 mg Puffersubstanz, bevorzugt mindestens 15 mg und ganz besonders bevorzugt mindestens 20 mg. Vorzugsweise sind maximal 48 mg und ganz besonders bevorzugt maximal 30 mg Puffersubstanz im Kern enthalten.

Der Kern kann mindestens einen weiteren pharmazeutisch annehmbaren Hilfsstoff enthalten wie beispielsweise Füll- und/oder Bindemittel. Der Kern kann zudem Sprengmittel enthalten, insbesondere ausgewählt aus Alginsäure, Calciumcarboxymethylcellulose, quervernetzter Carboxymethylcellulose, Hydroxypropylcellulose, Natriumcarboxymethylstärke, quervernetztem Polyvinylpyrrolidon und Mischungen davon. Der Zusatz eines solchen Sprengmittels verhindert, dass der Wirkstoffanteil aus dem Kern erst in zu tiefen Darmabschnitten freigesetzt wird. Dies würde mit einer deutlich schlechteren Resorption des Wirkstoffanteils einhergehen aufgrund der verminderten Resorptionsfläche. Als besonders geeignet hat sich ein Zusatz von quervernetztem Polyvinylpyrrolidon erwiesen.

Bevorzugt hat das Sprengmittel einen Gewichtsanteil am Kern von mindestens 1 Gew.-%, weiter bevorzugt mindestens 2 Gew.-%, um eine ausreichende Zerfallsförderung zu bewirken. Der Maximalgehalt an Sprengmittel liegt vorzugsweise bei 8 Gew.-%, bevorzugt bei maximal 6,5 Gew.-%. Zu hohe Anteile an Sprengmittel bewirken eine zu schnelle Freisetzung des Wirkstoffs. Alternativ ist der Kern vorzugsweise frei von solchen Sprengmitteln, da mit der erfindungsgemäß besonderen Kernzusammensetzung zumeist überraschenderweise bereits ein ausreichend schneller Zerfall gewährleistet ist.

Der Kern kann herstellungsbedingt mindestens ein Granuliermittel umfassen. Bevorzugt ist das Granuliermittel im Kern in einer Menge von mindestens 0,1 Gew.-%, weiter bevorzugt mindestens 0,25 Gew.-%, noch weiter bevorzugt mindestens 0,5 Gew.-% und noch weiter bevorzugt mindestens 0,75 Gew.-% enthalten. Maximal enthält der Kern vorzugsweise 5 Gew.-%, weiter bevorzugt maximal 2,5 Gew.-% Granuliermittel. Im Kern bevorzugte Granuliermittel sind weiter unten aufgeführt.

Herstellungsbedingt enthält der Kern vorzugsweise mindestens ein Gleitmittel, bevorzugt mit einem Masseanteil von höchstens 10 Gew.-%, bevorzugt höchstens 8 Gew.-% an der Gesamtmasse des Kerns. Vorzugsweise sind mindestens 0,5 Gew.-%, weiter bevorzugt mindestens 1 Gew.-% an Gleitmittel im Kern enthalten. Im Kern bevorzugte Gleitmittel sind weiter unten aufgeführt.

In einer Ausführungsform enthält der Kern also:
a. einen Wirkstoff; und
b. optional mindestens einen Trägerstoff (bevorzugt mit einem Masseanteil von 50 Gew.-% bis 90 Gew.-%), der insbesondere dazu dient, den Wirkstoff "zu tragen", dessen Hygroskopizität und Azidität abzuschwächen und auch eine gewisse Retardierung zu gewährleisten; und
c. optional mindestens eine Puffersubstanz (bevorzugt mit einem Masseanteil von 1 Gew.-% bis 26 Gew.-%) insbesondere zur Abschwächung der Azidität des Wirkstoffs; und
d. optional ein Sprengmittel (bevorzugter Masseanteil 1 Gew.-% bis 8 Gew.-%) zur Beschleunigung des Zerfalls des Kerns; und
e. optional herstellungsbedingt mindestens ein Granuliermittel (bevorzugter Masseanteil 0,1 Gew.-% bis 5 Gew.-%); und
f. optional herstellungsbedingt mindestens eine Gleitmittel (bevorzugter Masseanteil 0,5 Gew.-% bis 10 Gew.-%); und
g. optional weitere Hilfsstoffe wie beispielsweise Füll- und/oder Bindemittel.

Auf der Oberfläche des Kerns ist eine Zwischenschicht angeordnet. Eine solche Schicht hat sich als vorteilhaft erwiesen, um die Freisetzung des Wirkstoffs aus dem Kern und dem Mantel zeitlich zu versetzen. Vorzugsweise dient die Zwischenschicht auch dazu, Kern und Mantel aneinander zu binden. Ohne die Zwischenschicht konnte zumeist keine ausreichend bruchfeste und lagerfähige Arzneiform erreicht werden.

Außerdem hat es sich gezeigt, dass weniger Hilfsstoffe im Kern erforderlich sind, wenn die Zwischenschicht die Freigabe des Wirkstoffs aus dem Kern ausschließlich oder zusätzlich kontrolliert. Damit kann die Größe des Kerns reduziert werden und es wird eine angemessene Größe der gesamten Arzneiform sichergestellt, die für eine gute Patientencompliance notwendig ist. Auch erwies sich eine ausschließlich durch den Kern kontrollierte Freisetzung des Wirkstoffs insbesondere dann als schwierig, wenn es sich um einen hoch löslichen Wirkstoff handelt. So konnte die Freisetzung des Wirkstoffs aus dem Kern bei ausschließlicher Verwendung einer Retardmatrix häufig nicht ausreichend zeitlich von der Freisetzung des Wirkstoffs aus dem Mantel getrennt werden. Die Freisetzung konnte also nicht ausreichend lange aufrechterhalten werden. Erst mit der erfindungsgemäßen Zwischenschicht konnten die Freisetzung des Wirkstoffs aus dem Kern und aus dem Mantel ausreichend zeitlich versetzt werden.

Die Zwischenschicht ist vorzugsweise magensaftresistent ausgestaltet. "Magensaftresistent" bedeutet vorzugsweise, dass sich die Zwischenschicht nicht im pH-Bereich des Magens auflöst, sich also vorzugsweise nicht innerhalb von 120 Minuten in 0,1 N HCl bei 36°C bis 38°C auflöst. Dies bedeutet bevorzugt, dass der Kern, auf dessen Oberfläche sich die Zwischenschicht befindet, innerhalb von 120 Minuten in 0,1 N HCl bei 36 bis 38°C nicht zerfällt. Die Messung des Zerfalls erfolgt mit üblichen Zerfallsapparaturen, vorzugsweise der Blattrührerapparatur.

Die Freisetzung aus dem Kern beginnt also frühestens bei Eintritt in das Dünndarm-Milieu durch Auflösen, Zerfall, Reißen oder sonstige Veränderung der Zwischenschicht, bevorzugt durch Auflösen der Zwischenschicht bei Erreichen eines bestimmten pH-Wertes. Die Zwischenschicht löst sich also vorzugsweise bei Erreichen eines bestimmten pH-Wertes im wässrigen Milieu des Gastrointestinaltraktes auf. Die verschiedenen möglichen Prozesse werden erfindungsgemäß mit dem Begriff "Lösen" beschrieben. Das Lösen der Zwischenschicht erfolgt vorzugsweise ab einem pH-Wert von wenigstens 5, weiter bevorzugt ab einem pH-Wert von wenigstens 6 und ganz besonders bevorzugt ab einem pH-Wert von wenigstens 6,5. Ganz besonders bevorzugt löst sich die Zwischenschicht ab einem pH-Wert von wenigstens 7,0 und noch weiter bevorzugt ab einem pH-Wert von wenigstens 7,2. Ein Lösen bei zu niedrigen pH-Werten würde zu einer zu frühen Freisetzung des Wirkstoffs in der zweiten Phase noch im Magen oder in oberen Zwölffingerdarmabschnitten führen. Dies birgt je nach Wirkstoff die Gefahr zu hoher Plasmaspiegel. Die Zwischenschicht sollte sich vorzugsweise spätestens bei einem pH-Wert von 7,65, bevorzugt spätestens bei pH 7,5 lösen. Löst sich die Zwischenschicht erst bei zu hohen pH Werten, kann keine ausreichende Resorption des Wirkstoffs mehr gewährleistet werden.

Die Zwischenschicht umfasst vorzugsweise mindestens einen pharmazeutisch annehmbaren Hilfsstoff, insbesondere einen Filmbildner, der vorzugsweise die Magensaftresistenz sicherstellt. Optional kann auch ein Wirkstoff in der Zwischenschicht enthalten sein.

Als geeignete Filmbildner haben sich natürliche und synthetische Polymere erwiesen, die freie Säurefunktionen aufweisen. Bevorzugt sind diese Säurefunktionen in Salzform gut löslich. Bevorzugt umfasst jedes Polymer mindestens 0,1 freie Säurefunktionen je Monomerbaustein, weiter bevorzugt mindestens 0,3 freie Säureeinheiten je Monomerbaustein. Solche Polymere sind geeignet, sich im basischen pH-Bereich des Darms, nicht jedoch im sauren pH-Bereich des Magens aufzulösen. Bevorzugt sind solche freien Säurefunktionen Carboxylgruppen, da diese in Salzform zumeist ausreichend gut löslich sind.

Bevorzugte Filmbildner sind Cellulosederivate, Methacrylsäurepolymere, Polyvinylderivate und Mischungen davon.

Cellulosederivate sind vorzugsweise Cellulosen, die mit organischen Säuren verestert sind. Die organischen Säuren können aliphatisch oder aromatisch sein. Bevorzugt umfassen diese organischen Säuren höchstens 10 Kohlenstoffatome, weiter bevorzugt höchstens 8 Kohlenstoffatome. Sie umfassen bevorzugt aber mindestens 2 Kohlenstoffatome. Das Cellulosederivat ist besonders bevorzugt ausgewählt aus Celluloseacetat, Celluloseacetatphthalat, Celluloseacetattrimellitat, Hydroxypropylmethylcellulosephthalat, Hydroxypropylmethylcelluloseacetatsuccinat und Mischungen davon.

Bevorzugte Polyvinylderivate sind solche, die mit organischen Säuren verestert sind. Letztere können aliphatischer oder aromatischer Natur sein und umfassen bevorzugt mindestens 2 Kohlenstoffatome, vorzugsweise jedoch maximal 10 Kohlenstoffatome. Bevorzugt ist das Polyvinylderivat sowohl aliphatisch, als auch aromatisch verestert. Ein besonders bevorzugtes Polyvinylderivat ist Polyvinylacetatphthalat.

Unter den Methacrylsäurepolymeren haben sich solche als geeignet erwiesen, die aus Methacrylsäuremonomeren mit freien Carboxylgruppen und Monomeren mit veresterter Carboxylgruppe aufgebaut sind. Die Monomere mit veresterter Carboxylgruppe sind vorzugsweise ausgewählt aus Methacrylsäuremonomeren und Acrylsäuremonomeren. Die Monomere mit veresterter Carboxylgruppe sind vorzugsweise aliphatisch verestert, bevorzugt mit organischen Gruppen. Diese organischen Gruppen umfassen vorzugsweise mindestens 1 Kohlenstoffatom, vorzugsweise jedoch maximal 5 Kohlenstoffatome, weiter bevorzugt maximal 4 Kohlenstoffatome. Monomere mit veresterter Carboxylgruppe sind bevorzugt ausgewählt aus Methylmethacrylatmonomeren, Ethylacrylatmonomeren und Methylacrylatmonomeren.

Bevorzugte Methacrylsäurepolymere sind Polymethacrylsäure/Polymethylmethacrylat-Copolymere, Polymethacrylsäure/Polyethylacrylat-Copolymere, Polymethacrylsäure/Polymethylacrylat/Polymethylmethacrylat-Copolymere und Mischungen davon. Das Molverhältnis von Monomeren mit veresterten Carboxylgruppen zu Methacrylsäuremonomeren mit freier Carboxylgruppe beträgt dabei bevorzugt mindestens 0,5:1, besonders bevorzugt mindestens 0,9:1. Das Molverhältnis liegt vorzugsweise maximal bei 12:1, weiter bevorzugt bei maximal 10:1 und ganz besonders bevorzugt bei maximal 2,5:1. Ganz besonders bevorzugte Methacrylsäurepolymere sind Polymethacrylsäure/Polymethylmethacrylat-Copolymere. Besonders bevorzugt umfasst der Filmbildner wenigstens ein Methacrylsäurepolymer.

Besonders bevorzugte Filmbildner sind Eudragit^{®} L, Eudragit^{®} S, Eudragit^{®} L 100-55, Eudragit^{®} L30 D-55, Eudragit^{®} L 12,5, Eudragit^{®} S 12,5, Eudragit^{®} FS 30 D und Mischungen davon. Ganz besonders bevorzugt sind Eudragit^{®} L, Eudragit^{®} S und Mischungen davon, insbesondere Eudragit^{®} L 100, Eudragit^{®} S 100 und Mischungen davon. Eudragit^{®} L 100 entspricht Poly(methacrylsäure-co-methylmethacrylat) 1:1 (CAS 25086-15-1). Eudragit^{®} S 100 entspricht Poly(methacrylsäure-co-methylmethacrylat) 1:2 (CAS 25086-15-1). Eudragit^{®} L 100-55 entspricht Poly(methacrylsäure-co-ethylacrylat) 1:1 (CAS 25212-88-8). Eudragit^{®} L 30 D-55 entspricht Poly(methacrylsäureco-ethylacrylat) 1:1 (CAS 25212-88-8). Eudragit^{®} L 12,5 entspricht Poly(methacrylsäure-co-methylmethacrylat) 1:1 (CAS 25086-15-1). Eudragit^{®} S 12,5 entspricht Poly(methacrylsäure-co-methylmethacrylat) 1:2 (CAS 25086-15-1). Eudragit^{®} FS 30 D entspricht Poly(methylacrylat-co-methylmethacrylat-co-methacrylsäure) 7:3:1 (CAS 26936-24-3). In weiter bevorzugten Ausführungsformen werden Methacrylsäurepolymere verwendet, die Polyethylacrylat/Polymethylmethacrylat-Copolymere sind. Besonders bevorzugt ist Eudragit^{®} NE 30 D. Eudragit^{®} NE 30 D entspricht Poly(ethylacrylat-co-methylmethacrylat) 2:1 (CAS 9010-88-2).

Der Filmbildner ist ganz besonders bevorzugt ein Methacrylsäurepolymer, ganz besonders bevorzugt ist der Filmbildner wenigstens ein Polymethacrylsäure/Polymethylmethacrylat-Copolymer. In einer besonderen Ausführungsform werden ausschließlich Methacrylsäurepolymere als Filmbildner eingesetzt. Ganz besonders bevorzugt wird dabei eine Mischung von verschiedenen Methacrylsäurepolymeren eingesetzt. Durch die hohe Löslichkeit des Polymers im Darmmilieu kann eine gezielte Freisetzung des Wirkstoffs in der zweiten Phase vorzugsweise aus dem Kern im Dünndarm sichergestellt werden. Außerdem lassen sich diese Filmbildner kostengünstig verarbeiten und schnell und einfach auf die Oberfläche des Kerns auftragen. Durch Mischen verschiedener Methacrylsäurepolymere kann der pH-Wert, ab dem sich die Zwischenschicht lösen soll, gezielt eingestellt werden. Ganz besonders bevorzugt ist eine Mischung von Eudragit^{®} S 100 und Eudragit^{®} L 100. Das Masseverhältnis von Eudragit^{®} S 100 zu Eudragit^{®} L 100 beträgt dabei bevorzugt mindestens 1,5:1, vorzugsweise mindestens 2,5:1, noch weiter bevorzugt mindestens 3:1. Das Masseverhältnis liegt vorzugsweise bei maximal 10:1, bevorzugt maximal 7,5:1 und noch weiter bevorzugt bei maximal 5:1. In alternativen Ausführungsformen ist der Filmbildner Eudragit^{®} S 100 oder Eudragit^{®} L 100.

Alternativ kann ein natürliches Polymer als Filmbildner verwendet werden, bevorzugt ist Schellack.

Der Anteil des Filmbildners an der Zwischenschicht liegt vorzugsweise bei mindestens 20 Gew.-%, bevorzugt mindestens 35 Gew.-% und ganz besonders bevorzugt mindestens 40 Gew.-% sowie weiter bevorzugt mindestens 45 Gew.-%. Ein zu niedriger Gehalt an Filmbildner kann zu einer Auflösung der Zwischenschicht bereits im Magen führen. Der Gehalt des Filmbildners an der Zwischenschicht beträgt jedoch vorzugsweise maximal 75 Gew.-%, weiter bevorzugt maximal 70 Gew.-% und noch weiter bevorzugt maximal 65 Gew.-%. Ein zu hoher Gehalt führt zu einer sehr spröden Zwischenschicht ohne ausreichende Bindewirkung.

Das Gewicht der Zwischenschicht beträgt vorzugsweise wenigstens 4 mg, weiter bevorzugt wenigstens 5 mg und besonders bevorzugt mehr als 5 mg. Ganz besonders bevorzugt ist ein Gewicht der Zwischenschicht von wenigstens 8 mg und noch mehr bevorzugt von wenigstens 10 mg. Bei zu geringem Gewicht an Zwischenschicht kann die mechanische Stabilität des Kerns vermindert sein. Das Gewicht der Zwischenschicht beträgt vorzugsweise nicht mehr als 55 mg, weiter bevorzugt höchstens 50 mg und noch mehr bevorzugt höchstens 35 mg. Ganz besonders bevorzugt beträgt das Gewicht der Zwischenschicht nicht mehr als 28 mg. Bei zu hohem Gewicht, also zu hoher Menge an Zwischenschicht, besteht die Gefahr, dass die Freisetzung des Wirkstoffs aus dem Kern beeinträchtigt wird. Auch besteht die Gefahr, dass die Arzneiform insgesamt zu groß und zu schwer und damit schlechter schluckbar wird. Als ganz besonders geeignet hat sich ein Gewicht der Zwischenschicht von 12 bis 25 mg erwiesen.

Die Zwischenschicht ist geeignet, Kern und Mantel aneinander zu binden. Die Bindewirkung der Zwischenschicht wird bevorzugt durch den Zusatz von mindestens einem Weichmacher erreicht. Dies sind erfindungsgemäß bei Raumtemperatur und Normaldruck Flüssigkeiten. Besonders geeignet sind solche Weichmacher, die hygroskopisch sind. Besonders bevorzugt sind Polyalkohole mit mindestens zwei Hydroxylgruppen. Diese Polyalkohole enthalten bevorzugt mindestens 2 Kohlenstoffatome, vorzugsweise höchstens 10 Kohlenstoffatome. Die Hydroxylgruppen können frei vorliegen. Ebenso können alle oder ein Teil der Hydroxylgruppen verestert vorliegen, vorzugsweise mit organischen Säuren. Bevorzugt sind diese organischen Säuren aliphatische Säuren, weiter bevorzugt umfassen diese mindestens 2 Kohlenstoffatome, vorzugsweise höchstens 24 Kohlenstoffatome, am meisten bevorzugt höchstens 22 Kohlenstoffatome.

Ganz besonders bevorzugte Weichmacher sind ausgewählt aus Glycerol, Propylenglycol, Glyceroltriacetat, Rizinusöl, acetylierten Fettsäureglyceriden und Mischungen davon. Als ebenso erfindungsgemäß geeignet haben sich Polyether von Polyalkoholen erwiesen, wobei die Polyalkoholbausteine bevorzugt mindestens 2 Kohlenstoffatome und vorzugsweise maximal 10 Kohlenstoffatomen aufweisen. Bevorzugte Polyether sind Polyethylenglycole, vorzugsweise mit einer mittleren Molmasse bis 600 g/mol. Durch Zusatz solcher Weichmacher konnte eine gute Bindewirkung der Zwischenschicht erzielt werden. Außerdem sind diese Weichmacher nützlich, um die mechanische Stabilität auch über längere Zeiträume zu gewährleisten.

Der Anteil des Weichmachers an der Zwischenschicht beträgt bevorzugt mindestens 1 Gew.-%, bevorzugt mindestens 5 Gew.-%, um eine gute Bindewirkung zu erzielen. Bevorzugt beträgt der Anteil des Weichmachers an der Zwischenschicht mindestens 8 Gew.-% und weiter bevorzugt mindestens 12 Gew.-%. Vorzugsweise sind maximal 30 Gew.-%, weiter bevorzugt maximal 25 Gew.-% und noch weiter bevorzugt maximal 20 Gew.-% an Weichmacher in der Zwischenschicht enthalten. Bei zu hohen Gehalten an Weichmacher kann keine ausreichende Festigkeit der Zwischenschicht sowie ausreichende Magensaftresistenz mehr sichergestellt werden.

Die Zwischenschicht kann mindestens einen weiteren pharmazeutisch annehmbaren Hilfsstoff umfassen, der die Verarbeitung und Auftragung der Schicht vereinfachen kann und/oder zur Einstellung von deren Konsistenz geeignet ist. Insbesondere können übliche Füllmittel, Porenbildner und/oder weitere Weichmacher enthalten sein. Weitere Weichmacher können Ester von organischen Säuren sein, bevorzugt umfassen diese organischen Säuren mindestens 2 Kohlenstoffatome und vorzugsweise höchstens 10 Kohlenstoffatome. Bevorzugt sind Citronensäure und Phthalsäure. Solche Weichmacher sind beispielsweise Tributylcitrat, Triethylcitrat, Diethylphthalat, Dibutylphthalat.

Um die Auftragung der Zwischenschicht zu erleichtern, enthält die Zwischenschicht vorzugsweise Füllstoffe, bevorzugt ausgewählt aus Talkum, Titandioxid, Magnesiumstearat, Farbstoffen, Glycerolmonostearat, Lactose, mikrokristalline Cellulose, Polyvinylpyrrolidon und Mischungen daraus. Vorzugsweise ist der Füllstoff Talkum. Besonders bevorzugt ist der Füllstoff zur Perforation der Zwischenschicht geeignet, so dass die Freisetzung des Wirkstoffs im Kern zusätzlich gesteuert werden kann. Allerdings ist der Massenanteil des Füllstoffes auf vorzugsweise höchstens 40 Gew.-%, weiter bevorzugt höchstens 35 Gew.-% zu begrenzen, insbesondere wenn die Zwischenschicht eine Bindewirkung haben soll. Der Füllstoff kann die Bindewirkung der Zwischenschicht mindern. Der Mindestgehalt an Füllstoff beträgt bevorzugt mindestens 5 Gew.-%, weiter bevorzugt mindestens 15 Gew.-%.

Das Masseverhältnis von Kern zu Zwischenschicht sollte vorzugsweise mindestens 2:1 betragen, weiter bevorzugt wenigstens 3:1 betragen, noch weiter bevorzugt mindestens 6,5:1 und ganz besonders bevorzugt mindestens 7,5:1. Die Zwischenschicht sollte also im Verhältnis zum Kern keinen zu hohen Masseanteil aufweisen, insbesondere wenn die Zwischenschicht keinen Wirkstoff umfasst. Damit wird sichergestellt, dass auch das Gesamtgewicht und damit die Abmessungen der Arzneiform für die orale Einnahme geeignet sind. Das Masseverhältnis sollte vorzugsweise höchstens 60:1, weiter bevorzugt höchstens 45:1 betragen. In einer bevorzugten Ausführungsform beträgt das Masseverhältnis höchstens 40:1 und noch weiter bevorzugt höchstens 30:1 sowie insbesondere bevorzugt höchstens 20:1. Nimmt das Verhältnis zu hohe Werte an, ist die Zwischenschicht für die gewünschte Funktionalität nicht mehr ausreichend dick.

Die Zwischenschicht bedeckt vorzugsweise mindestens 40% der Gesamtoberfläche des Kerns. Dies ist insbesondere dann vorteilhaft, wenn der Kern an der nicht bedeckten Fläche mit einer anderen Schicht bedeckt ist. Besonders bevorzugt ist mindestens 95% der Gesamtoberfläche des Kerns von der Zwischenschicht bedeckt, weiter bevorzugt mindestens 98% und besonders bevorzugt mindestens 99%. In besonders bevorzugten Ausführungsformen ist der Kern vollständig von der Zwischenschicht bedeckt. Die Zwischenschicht hat in einer bevorzugten Ausführungsform vorzugsweise keine Poren. Mit "Poren" sind Öffnungen in der Zwischenschicht gemeint, durch die der Wirkstoff aus dem Kern verfrüht austreten könnte.

In einer Ausführungsform enthält die Zwischenschicht also:
a. einen Wirkstoff,
b. optional einen Filmbildner (bevorzugter Masseanteil 20 bis 75 Gew.-%), insbesondere zur Gewährleistung einer Magensaftresistenz der Zwischenschicht, und
c. optional einen Weichmacher (bevorzugter Masseanteil 1 bis 30 Gew.-%), der bevorzugt hygroskopisch ist, insbesondere um eine gute Bindewirkung der Zwischenschicht zu gewährleisten; und
d. optional einen Füllstoff, insbesondere Talkum in einem bevorzugten Masseanteil von 5 Gew.-% bis 40 Gew.-%; und
e. optional weitere pharmazeutisch annehmbare Hilfsstoffe wie beispielsweise weitere Füllstoffe, Porenbildner und/oder weitere Weichmacher.

Der Mantel ist wirkstoffhaltig, enthält also mindestens den Wirkstoff. Neben dem Wirkstoff ist vorzugsweise mindestens ein pharmazeutisch annehmbarer Hilfsstoff enthalten.

Das Masseverhältnis von Mantel zu Kern beträgt vorzugsweise wenigstens 0,5:1, weiter bevorzugt wenigstens 1,1:1, mehr bevorzugt wenigstens 1,8:1, weiter bevorzugt wenigstens 2:1 und besonders bevorzugt wenigstens 2,2:1. Dies ermöglicht eine ausreichende Verarbeitbarkeit des Mantels. Das Masseverhältnis beträgt vorzugsweise maximal 10:1, bevorzugt maximal 5:1, bevorzugt maximal 4,5:1 und weiter bevorzugt maximal 4:1. Bei zu hohen Masseverhältnissen kann eine für die Einnahme geeignete Größe der Arzneiform nicht mehr sichergestellt werden. Vorzugsweise enthält der Mantel einen vergleichsweise höheren Anteil pharmazeutisch annehmbarer Hilfsstoffe als der Kern, um ein bestimmtes Volumen des Mantels sicherzustellen sowie auch um den Wirkstoff einzubetten und dabei ausreichend nach außen beispielsweise vor Feuchtigkeit und insbesondere auch Licht abzuschirmen. Das Masseverhältnis der Hilfsstoffe im Mantel zum Hilfsstoffanteil im Kern liegt bevorzugt bei mindestens 1,8:1, weiter bevorzugt sogar bei mindestens 2,5:1.

Insbesondere hat sich auch im Mantel die Verwendung mindestens eines Trägerstoffs als Hilfsstoff als vorteilhaft erwiesen. Vorzugsweise ist der Trägerstoff im Mantel hygroskopisch. Es wäre zu erwarten gewesen, dass dies die Stabilität der Arzneiform beeinträchtigt, zumal der Mantel bevorzugt keinen weiteren Überzug aufweist und insofern die Mantelbestandteile nicht von Feuchtigkeit und Licht abgeschirmt sind. Das Masseverhältnis des Trägerstoffs im Mantel zum Wirkstoff im Mantel beträgt vorzugsweise mindestens 7,5:1, bevorzugt mindestens 10:1, weiter bevorzugt mindestens 14:1 und in ganz besonders bevorzugten Ausführungsformen mindestens 15:1. Der Wirkstoff liegt dann vorzugsweise verteilt und matrixartig eingebettet im verhältnismäßig größeren Trägerstoffanteil im Mantel vor, so dass eine weitere Wasseraufnahme aus der Umgebung deutlich vermindert werden kann. Das Masseverhältnis beträgt jedoch vorzugsweise maximal 50:1, weiter bevorzugt maximal 35:1, um eine geeignete Größe der Arzneiform sicherzustellen. Weiter bevorzugt beträgt das Masseverhältnis maximal 30:1 und noch weiter bevorzugt maximal 28:1.

Geeignete Trägerstoffe im Mantel können anorganischer oder organischer Art sein. Bevorzugte Trägerstoffe im Mantel sind ausgewählt aus natürlichen und synthetischen Polymeren. Bevorzugt handelt es sich dabei um natürliche oder modifizierte Polysaccharide, vorzugsweise bestehend aus zwei oder mehreren gleichen oder verschiedenen Monosaccharidbausteinen. Solche Stoffe sind kostengünstig erhältlich und leicht verarbeitbar. Als besonders vorteilhaft haben sich Polysaccharide erwiesen, die Glucoseeinheiten enthalten. Der Trägerstoff ist bevorzugt ein Disaccharid, besonders bevorzugt ist dieses ausgewählt aus Lactose, Saccharose und Mischungen davon.

Alternativ kann es sich bei dem Trägerstoff im Mantel um ein Polysaccharid mit mehr als 10 Monosaccharidbausteinen handeln, das vorzugweise bei Kontakt mit Wasser quellfähig ist. Besonders bevorzugt handelt es sich dabei um Stärke einschließlich von Stärkederivaten, Cellulose einschließlich von Cellulosederivaten oder Mischungen davon. Ganz besonders bevorzugt ist pulverisierte Cellulose wie mikrokristalline Cellulose. Ebenso kann Stärke eingesetzt werden in nativer oder vorverkleisterter Form, insbesondere Maisstärke. Solche Trägerstoffe sind noch besser in der Lage, Wasser von dem Wirkstoffanteil aufzunehmen. Allerdings zeigte sich, dass die Bruchfestigkeit der Tablette bei ausschließlicher Verwendung solcher Polysaccharide als Trägerstoffe im Mantel bei Verwendung bestimmter Wirkstoffe mit zunehmender Lagerdauer sinken kann.

In einer besonders bevorzugten Ausführungsform sind daher mindestens zwei Trägerstoffe im Mantel enthalten, ganz besonders bevorzugt handelt es sich dabei um mindestens ein Disaccharid und um mindestens ein Polysaccharid mit mehr als 10 Monosaccharidbausteinen, das vorzugsweise bei Kontakt mit Wasser quellfähig ist. Durch diese Kombination wurde eine optimale Bruchfestigkeit und Stabilität erzielt. Auch ist der Wirkstoffanteil vorzugsweise auf dem Trägerstoffgemisch optimal fixiert. Ganz besonders bevorzugt enthält der Mantel mindestens drei Trägerstoffe. Das Masseverhältnis von Polysacchariden mit mehr als 10 Monosaccharidbausteinen zu Disacchariden beträgt vorzugsweise mindestens 1,1:1, bevorzugt mindestens 1,25:1 und ganz bevorzugt mindestens 1,3:1.

Es können auch anorganische Trägerstoffe, vorzugsweise mit hygroskopischen Eigenschaften im Mantel eingesetzt werden. Anorganische Trägerstoffe können auch zusätzlich zu den oben genannten Trägerstoffen im Mantel enthalten sein.

Bevorzugt umfasst der Mantel Salze der oben genannten organischen oder anorganischen Substanzen in einem Anteil von maximal 5 Gew.-%, bevorzugt maximal 2 Gew.-% und ganz besonders bevorzugt in einem Anteil von maximal 1 Gew.-%. Werden Salze der oben genannten organischen Substanzen oder Salze anorganischer Substanzen im erfindungsgemäßen Mantel eingesetzt, kann es zur Reaktion des Wirkstoffs im Mantel mit diesen Substanzen kommen, wodurch die Stabilität des Wirkstoffs im Mantel nachteilig beeinflusst werden kann. Salze umfassen dabei insbesondere Alkali- und Erdalkalisalze solcher Substanzen. Besonders bevorzugt ist der Mantel frei von Salzen der oben genannten organischen oder anorganischen Substanzen, d.h. Salze solcher Substanzen, insbesondere umfassend Calciumphosphat, Natriumsalze von Cellulosen und Magnesiumstearat, sind lediglich als Verunreinigungen in einem Anteil von vorzugsweise maximal 0,5 Gew.-%, bevorzugt von maximal 0,1 Gew.-% an der Gesamtmasse des Mantel enthalten. Jedoch können Salze solcher Substanzen in einem optionalen Überzug des Mantels enthalten sein.

Besonders bevorzugt sind mindestens zwei Trägerstoffe im Mantel enthalten, ganz besonders bevorzugt drei Trägerstoffe. Bevorzugt beträgt der Gesamtanteil von Trägerstoffen an der Gesamtmasse des Mantels mindestens 70 Gew.-%, bevorzugt mindestens 77,5 Gew.-% und ganz besonders bevorzugt mindestens 80 Gew.-% um die Hygroskopizität des Wirkstoffs im Mantel ausreichend zu kompensieren und gleichzeitig eine gute Verarbeitbarkeit des Mantels sicherzustellen. Der Mantel enthält vorzugsweise maximal 97 Gew.-% Trägerstoff, weiter bevorzugt maximal 95 Gew.-%. Zu hohe Mengen an Trägerstoff erschweren die Verarbeitbarkeit des Mantels.

Bevorzugt enthält der Mantel eine Puffersubstanz. Als Puffersubstanzen sind organische Säuren besonders gut geeignet, vorzugsweise sind diese niedermolekular. Als "niedermolekular" sind organische Säuren zu bezeichnen, die Molmassen unter 300 g/mol aufweisen. Die Puffersubstanz im Mantel ist bevorzugt eine Carbonsäure ausgewählt aus Citronensäure, Milchsäure, Weinsäure, Fumarsäure, Maleinsäure und Ascorbinsäure sowie Mischungen daraus. Besonders bevorzugt sind alpha-Hydroxycarbonsäuren, die aufgrund der alpha-Hydroxygruppe eine optimale Pufferwirkung zeigen. Bevorzugt sind diese ausgewählt aus Milchsäure, Weinsäure, Citronensäure und Mischungen davon. Ganz besonders vorteilhaft ist Citronensäure, die sich besonders leicht verarbeiten lässt und die Freisetzung des Wirkstoffs aus dem Mantel dabei nicht signifikant verändert. Dies ist überraschend, da Citronensäure in vielen Darreichungsformen eine Freisetzungsbeschleunigung bewirkt, was hier unerwünscht wäre.

Das Masseverhältnis des Wirkstoffs im Mantel zur Puffersubstanz im Mantel liegt vorzugsweise bei mindestens 0,5:1, bevorzugt mindestens 0,8:1 und weiter bevorzugt bei mindestens 0,9:1. Dieses Masseverhältnis beträgt vorzugweise maximal 1,5:1, weiter bevorzugt maximal 1,2:1 und besonders bevorzugt maximal 1,1:1. Bei zu geringer Menge an Puffersubstanz kann die Azidität des Wirkstoffs im Mantel nicht ausreichend gepuffert werden. Bei zu hoher Menge an Puffersubstanz nimmt die Azidität wiederrum unvorteilhaft zu. Der Mantel enthält vorzugsweise mindestens 0,5 Gew.-%, weiter bevorzugt mindestens 1 Gew.-% und noch weiter bevorzugt wenigstens 2,5 Gew.-% Puffersubstanz. Bevorzugt enthält der Mantel maximal 15 Gew.-%, weiter bevorzugt maximal 10 Gew.-% und noch weiter bevorzugt maximal 8 Gew.-% an Puffersubstanz.

Überraschenderweise erwies sich Citronensäure als Puffersubstanz auch für den Mantel als besonders geeignet. So konnte eine ausreichende Abschwächung der Azidität erreicht werden, ohne dass die Freisetzung des Wirkstoffanteils aus dem Mantel erkennbar beschleunigt wurde. So war eigentlich zu erwarten, dass die Puffersubstanz die Freisetzung des Wirkstoffs beschleunigt. Der Mantel enthält erfindungsgemäß bevorzugt mindestens 8 mg Puffersubstanz, bevorzugt mindestens 15 mg und ganz besonders bevorzugt mindestens 20 mg. Vorzugsweise sind maximal 48 mg und ganz besonders bevorzugt maximal 30 mg Puffersubstanz im Mantel enthalten.

Der Mantel kann mindestens einen weiteren pharmazeutisch annehmbaren Hilfsstoff enthalten wie beispielsweise Füll- und Bindemittel. Bevorzugt ist jedoch kein Sprengmittel im Mantel enthalten, dies könnte zu einer zu schnellen Wirkstofffreisetzung aus dem Mantel führen.

Der Mantel kann herstellungsbedingt mindestens ein Granuliermittel umfassen. Bevorzugt ist das Granuliermittel im Mantel in einer Menge von mindestens 0,02 Gew.-%, weiter bevorzugt mindestens 0,1 Gew.-% enthalten. Bevorzugt enthält der Mantel maximal 2,5 Gew.-% an Granuliermittel. Im Mantel bevorzugte Granuliermittel sind weiter unten aufgeführt.

Herstellungsbedingt enthält der Mantel vorzugsweise mindestens ein Gleitmittel, bevorzugt mit einem Masseanteil von höchstens 5 Gew.-%, weiter bevorzugt höchstens 4,5 Gew.-%. Vorzugsweise sind mindestens 0,25 Gew.-%, weiter bevorzugt mindestens 0,5 Gew.-% an Gleitmittel im Mantel enthalten. Im Mantel bevorzugte Gleitmittel sind weiter unten aufgeführt.

Der Mantel bedeckt vorzugsweise mindestens 40% der Gesamtoberfläche der dem Kern gegenüberliegenden Seite der Zwischenschicht. Ein Teil der Fläche kann dann völlig unbedeckt bleiben, um beispielsweise die Größe der Arzneiform zu reduzieren, oder wenn die Fläche von einer anderen Schicht bedeckt sein soll. Besonders bevorzugt ist mindestens 95% der Gesamtoberfläche vom Mantel bedeckt, weiter bevorzugt mindestens 98% und besonders bevorzugt mindestens 99%. In einer bevorzugten Ausführungsform ist die Zwischenschicht vollständig von dem Mantel bedeckt.

Bevorzugt enthält der Mantel selbst keinen weiteren Überzug, insbesondere ist kein Überzug auf dem Mantel vorhanden, der die Freisetzung des Wirkstoffs verzögert oder verlängert. Es ist also vorzugsweise kein Film auf den Mantel aufgetragen. In einer alternativen Ausführungsform kann jedoch ein schnellzerfallender Überzug auf dem Mantel vorteilhaft sein, der vorzugsweise im wässrigen Milieu des Magens schnell zerfällt. Dieser enthält bevorzugt wasserlösliche Polymere als Filmbildner wie Cellulosederivate, Polyvinylpyrrolidon, Polyvidonacetat. Cellulosederivate sind bevorzugt ausgewählt aus Methylcellulose, Hydroxypropylcellulose, Hydroxypropylmethylcellulose, Natriumcarboxymethylcellulose und Mischungen davon. Alternativ kann der Mantel mit einem zuckerhaltigen Dragiersirup überzogen sein.

In einer Ausführungsform enthält der Mantel also:
a. einen Wirkstoff; und
b. optional mindestens einen Trägerstoff (bevorzugt mit einem Masseanteil von 70 Gew.-% bis 97 Gew.-%), der insbesondere dazu dient, den Wirkstoff "zu tragen" und einzubetten, dessen Hygroskopizität und Azidität abzuschwächen und auch eine gewisse Retardierung zu gewährleisten; und
c. optional mindestens eine Puffersubstanz (bevorzugt mit einem Masseanteil von 0,5 Gew.-% bis 15 Gew.-%) insbesondere zur Abschwächung der Azidität des Wirkstoffs; und
d. optional herstellungsbedingt mindestens ein Granuliermittel (bevorzugter Mengenbereich 0,02 Gew.-% bis 2,5 Gew.-%); und
e. optional herstellungsbedingt mindestens ein Gleitmittel (bevorzugter Mengenbereich 0,25 Gew.-% bis 5 Gew.-%); und
f. optional weitere Hilfsstoffe wie beispielsweise Füll- und Bindemittel.

Die Erfindung betrifft auch ein Herstellungsverfahren für die erfindungsgemäße Arzneiform. Dieses umfasst die Schritte:
a) Herstellen des Kerns,
b) Herstellen der Zwischenschicht,
c) Herstellen des Mantels.

Ein direktes Verpressen des Wirkstoffs vorzugsweise zusammen mit Hilfsstoffen ist wegen der typischerweise vorliegenden Hygroskopizität des Wirkstoffs oft schwierig. Ein Verpressen von Granulaten führt zu einem Komprimat mit guter Festigkeit und vor allem geringem Abrieb. Außerdem stellt die Bereitstellung der zu verpressenden Mischung als Granulat üblicherweise erst eine ausreichende Haftfähigkeit und Fließfähigkeit sicher. Die Herstellung des Kerns umfasst also bevorzugt die Herstellung eines Wirkstoffgranulats aus dem Wirkstoff und vorzugsweise den für den Kern bevorzugten Hilfsstoffen. Die direkte Herstellung des Wirkstoffgranulats war jedoch durch die Hygroskopizität des Wirkstoffs erheblich erschwert. Es war insbesondere schwierig mit üblichen Granulierverfahren ausreichend trockene und stabile Wirkstoffgranulate zu erhalten. Häufig waren die erhaltenen Wirkstoffgranulate zu feucht, so dass es zu einer ungenügenden Festigkeit, Dosierungsschwankungen und Deckeln des Kerns beispielsweise beim Pressen kam.

Als vorteilhaft erwies sich überraschenderweise eine kombinierte Granulation. Diese umfasst erfindungsgemäß zunächst das Herstellen eines Vorgranulats, das erfindungsgemäß wirkstofffrei ist. Das Vorgranulat umfasst vorzugsweise den Trägerstoff des Kerns und die Puffersubstanz des Kerns sowie gegebenenfalls weitere Hilfsstoffe. Aus dem Vorgranulat wird anschließend das Wirkstoffgranulat hergestellt.

Diese bevorzugte kombinierte Granulation erwies sich als insgesamt schonender für den Wirkstoff. Auch konnten überraschenderweise feste und stabile Kerne ohne nennenswerte Dosisschwankungen hergestellt werden. Ohne nennenswerte Dosisschwankungen bedeutet dabei, dass der Gehalt des Wirkstoffs im Kern, gemessen über 5 Kerne, vorzugsweise bei mindestens 93 %, bevorzugt bei mindestens 95% liegt und vorzugsweise maximal bei 108%, weiter bevorzugt bei maximal 107% der theoretischen Wirkstoffmenge je Kern. Dies überrascht, da eine kombinierte Granulation generell mit der Gefahr einer ungleichen Wirkstoffverteilung und einer erhöhten Entmischungstendenz verbunden ist und daher üblicherweise vermieden wird.

Zur Herstellung des Vorgranulats hat sich die Feuchtgranulierung als vorteilhaft erwiesen, besonders bevorzugt die Herstellung eines Klebstoffgranulats. Das Herstellen des Vorgranulats umfasst also vorzugsweise den Schritt des Mischens von Trägerstoff und vorzugsweise Puffersubstanz mit einer Granulierlösung. Überraschenderweise kann mit einem solchen Verfahren ein stabiles Vorgranulat mit geringer Restfeuchte erhalten werden. Da der Trägerstoff typischerweise hygroskopisch ist, wäre eigentlich davon auszugehen, dass eine geringe Restfeuchte des Klebstoffgranulats nur mit erheblichem Aufwand bei hohen Trocknungstemperaturen erzielt werden kann, was wiederrum kostenintensiv ist.

Bevorzugt umfasst die Granulierlösung eine wässrige Lösung eines Granuliermittels. Das Granuliermittel ist ein Stoff mit klebenden und kleisternden Eigenschaften. Dabei handelt es sich vorzugsweise um synthetische und/oder natürliche Polymere. Bevorzugt ist das Granuliermittel ausgewählt aus Stärken, Polyvinylpyrrolidon, Gelatine, Celluloseethern und Mischungen davon. Ganz besonders bevorzugt wird Polyvinylpyrrolidon eingesetzt, da eine besonders gute Klebwirkung bei Verwendung hygroskopischer Wirkstoffe erzielt werden konnte. Allerdings ist das Polyvinylpyrrolidon vorzugsweise derart zu wählen, dass die mittlere Molmasse 40.000 g/mol nicht überschreitet. Zu hohe Molekulargewichte sind mit einer hohen Viskosität der Granulierlösung verbunden, so dass das Granulieren deutlich erschwert wird. Insbesondere geeignet ist Povidon K25.

Die Herstellung des Vorgranulats umfasst vorzugsweise einen Trocknungsschritt. Hierbei kommen Trocknungsverfahren wie Sprühtrocknung, Wirbelschichttrocknung, Vakuumtrocknung und/oder Gefriertrocknung in Frage. Bevorzugt ist die Wirbelschichttrocknung, vorzugsweise bei einer Zulufttemperatur von höchstens 75°C, weiter bevorzugt höchstens 65°C. Die Restfeuchte des Vorgranulats wird bevorzugt auf kleiner 8 Gew.-% eingestellt, weiter bevorzugt auf kleiner als 5 Gew.-%. Hat das Vorgranulat eine zu hohe Restfeuchte, so führt dies zu einer ungenügenden Kernfestigkeit. Außerdem sind Dosisschwankungen zu beobachten.

Das Herstellen des Wirkstoffgranulats umfasst vorzugsweise das Mischen des Vorgranulats mit einer Wirkstofflösung. Dabei wird der Wirkstoff an das Vorgranulat gebunden. Die Wirkstofflösung umfasst vorzugsweise den Wirkstoff und ein Lösungsmittel. Das Lösungsmittel ist bevorzugt ein Alkohol, besonders bevorzugt ein aliphatischer Alkohol. Vorzugsweise ist der aliphatische Alkohol ausgewählt aus Methanol, Ethanol, n-Propanol, *iso*-Propanol und Mischungen davon. Methanol hat dabei ein gutes Lösungsvermögen bei geringem Siedepunkt und ist daher besonders bevorzugt. Allerdings hat die Verwendung von Methanol den Nachteil, dass etwaige Rückstände aufgrund der Toxizität nahezu vollständig entfernt werden müssen.

Das Verfahren zur Herstellung des Wirkstoffgranulats umfasst vorzugsweise einen Trocknungsschritt. Insbesondere die Wirbelschichttrocknung hat sich dabei als besonders vorteilhaft erwiesen, da eine rasche Trocknung erzielt werden konnte. Erfindungsgemäß konnte dadurch das Lösungsmittel vollständig aus dem Wirkstoffgranulat entfernt werden. Unter einer vollständigen Entfernung des Lösungsmittels ist erfindungsgemäß ein Restlösungsmittelgehalt unter 5000 ppm (m/m), weiter bevorzugt von höchstens 3.000 ppm (m/m), ganz besonders bevorzugt liegt der Restlösungsmittelgehalt unter 3.000 ppm (m/m) bezogen auf die Gesamtmasse des getrockneten Wirkstoffgranulats.

Die Herstellung des Kerns umfasst erfindungsgemäß bevorzugt das Verpressen des Wirkstoffgranulats. Das

Verpressen kann beispielsweise mit einer Rundläuferpresse, Exzenterpresse oder sonstigen Tablettiereinrichtungen erfolgen. Allerdings kann dabei der Kern am Stempel kleben und deckeln. Vorzugsweise wird das Wirkstoffgranulat daher mit mindestens einem Gleitmittel vermischt, um eine verpressbare Kernmischung zu erhalten. Das Vorgehen hat sich als vorteilhaft erwiesen und es können feste Kerne mit gutem Zerfall erhalten werden. Dies überrascht, da eine Entmischungstendenz bei Vermischung des hoch kompakten Wirkstoffgranulats mit dem Gleitmittel zu erwarten gewesen wäre. Der Zusatz des Gleitmittels bereits bei der Herstellung des Vorgranulats und/oder Wirkstoffgranulats kann zu weniger festen und schlechter verpressbaren Granulaten führen. Daher erfolgt der Zusatz des Gleitmittels vorzugsweise erst nach Herstellung des Wirkstoffgranulats.

Insbesondere Fettsäurederivate haben sich als vorteilhafte Gleitmittel erwiesen. Fettsäurederivate umfassen erfindungsgemäß Salze von Fettsäuren, Fettsäureester, Fettsäuren und Fette sowie Mischungen daraus, wobei die Fettsäuren bevorzugt mindestens 8 Kohlenstoffatome umfassen, weiter bevorzugt mindestens 12 Kohlenstoffatome. Dagegen kann die Verwendung von Talkum als Gleitmittel im erfindungsgemäßen Verfahren oft zu einer suboptimalen Gleitwirkung führen. Besonders vorteilhaft ist die Verwendung von Fetten, die bei Raumtemperatur flüssig sind und damit eine einfache Verarbeitung zulassen. Insbesondere bevorzugt ist dabei Baumwollsamenöl. Bei Verwendung solcher Fette werden Kerne mit optimaler Bruchfestigkeit erhalten, die nicht zum Deckeln neigen.

Das Gleitmittel wird in einer solchen Menge eingesetzt, dass ein Masseanteil von höchstens 10 Gew.-%, bevorzugt höchstens 8 Gew.-% an der Gesamtmasse der Mischung aus Wirkstoffgranulat und Gleitmittel, also der Kernmischung, nicht überschritten wird. Zu hohe Mengen an Gleitmittel verringern die Benetzbarkeit des Kerns und können damit den Zerfall des Kerns negativ beeinflussen. Um eine ausreichende Gleitwirkung zu erzielen, sollten vorzugsweise mindestens 0,5 Gew.-%, bevorzugt mindestens 1 Gew.-% an Gleitmittel an der Gesamtmasse der Kernmischung verwendet werden, die dann entsprechend auch im Kern enthalten sind.

Der erhaltene Kern hat bevorzugt ein Gewicht von maximal 500 mg, bevorzugt maximal 350 mg, weiter bevorzugt maximal 250 mg. Der Durchmesser des Kerns liegt vorzugsweise bei maximal 12 mm, weiter bevorzugt maximal 10 mm und besonders bevorzugt bei maximal 9 mm. Bei zu großen Kernen ist die Weiterverarbeitung erschwert und es werden zumeist Arzneiformen erhalten, die nur schwer zu schlucken sind.

Die Herstellung der Zwischenschicht umfasst vorzugsweise das Lösen des Filmbildners in einem Lösungsmittel. Ebenfalls umfasst das Herstellen der Zwischenschicht vorzugsweise die Zugabe der gegebenenfalls weiteren Hilfsstoffe, insbesondere des Weichmachers. Es resultiert eine Zwischenschichtmischung.

Das Lösungsmittel zum Lösen des Filmbildners ist bevorzugt ein aliphatischer Alkohol ausgewählt aus Methanol, Ethanol, n-Propanol, *iso*-Propanol und Mischungen davon. Am meisten bevorzugt ist *iso*-Propanol. Die Zwischenschichtmischung wird auf die Oberfläche des Kerns aufgetragen. Als Verfahren kommen hierfür beispielsweise Kesselverfahren in Dragierkesseln, Trommelkesseln, GS-Coatern, sowie Tauchrohrverfahren oder Wirbelschichtverfahren in Frage. Besonders bevorzugt werden GS-Coater verwendet, da diese eine schnellere und gleichmäßigere Beschichtung des Kerns zulassen als beispielsweise übliche Dragierkessel. Das Auftragen der Zwischenschichtmischung erfolgt dabei vorzugsweise bei einer Zulufttemperatur von höchstens 75°C, weiter bevorzugt höchstens 60°C. Zu hohe Temperaturen können zur Zersetzung des Wirkstoffs im Kern und gegebenenfalls der Zwischenschicht führen.

Die Herstellung des Mantels umfasst vorzugsweise das Herstellen eines Wirkstoffgranulats. Vorzugsweise ist dieses wie das Wirkstoffgranulat für den Kern hergestellt. Da das direkte Verpressen des Wirkstoffgranulats nur schwer möglich ist und um ein geeignetes Volumen des Mantels sicherzustellen, wird das Wirkstoffgranulat vorzugsweise mit mindestens einem pharmazeutisch annehmbaren Hilfsstoff vermischt, so dass eine Mantelmischung erhalten wird. Vorzugsweise ist der mindestens eine Hilfsstoff ein Trägerstoff im Mantel.

Dabei hat sich ein Masseanteil des Wirkstoffgranulats am Mantel von vorzugsweise mindestens 10 Gew.-%, bevorzugt mindestens 20 Gew.-% als vorteilhaft erwiesen. Der Masseanteil des Granulats liegt vorzugsweise bei maximal 49 Gew.-%, bevorzugt maximal 42 Gew.-%.

Bei einem solchen Vorgehen wäre eigentlich eine starke Entmischungstendenz zu erwarten zwischen dem vergleichsweise hohen Hilfsstoffanteil und dem kompakten Wirkstoffgranulat, mit der Folge eines schlechten Zerfalls. Überraschenderweise kann aber mit diesem Vorgehen nun gerade ein stabiler Mantel mit guten Zerfallseigenschaften erhalten werden.

Die Herstellung der erfindungsgemäßen Arzneiform umfasst vorzugsweise das Auftragen der Mantelmischung auf die Zwischenschicht. Der Begriff "Auftragen" umfasst erfindungsgemäß verschiedenste Verfahren, vorzugsweise das Pressen des Mantels auf die Zwischenschicht bevorzugt unter Verwendung üblicher Tablettenpressen. Damit es nicht zum Deckeln, also der Abstoßung einzelner gepresster Schichten, kommt und damit das Kleben an den Stempeln verhindert wird, wird der Mantelmischung bevorzugt mindestens ein Gleitmittel zugesetzt

Bevorzugte Gleitmittel entsprechen denen, die bei der Herstellung des Kerns verwendet werden. Es hat sich aber gezeigt, dass bereits sehr geringe Anteile an Gleitmittel für eine gute Pressbarkeit der Mantelmischung ausreichend sind und eine Steigerung der Menge überraschenderweise schnell die Benetzbarkeit und den Zerfall des Mantels negativ beeinflusst. Daher ist das Gleitmittel im Mantel vorzugsweise auf 5 Gew.-%, bevorzugt 4,5 Gew.-% zu begrenzen. Als vorteilhafte Gleitmittel haben sich insbesondere Fette erwiesen, die bei Raumtemperatur und Normaldruck flüssig sind und sich somit leicht verarbeiten lassen. Ganz besonders bevorzugt wird Baumwollsamenöl verwendet.

Das Aufpressen des Mantels auf die Zwischenschicht erfolgt bevorzugt derart, dass die Mantelmischung in die Presse vorgelegt wird. Soll sich der Kern mit der Zwischenschicht etwa in der Mitte der herzustellenden Arzneiform befinden, wird bevorzugt mindestens 40 Gew.-%, weiter bevorzugt mindestens 45 Gew.-% der Mantelmischung in eine Matrize vorgelegt. Es werden aber vorzugsweise höchstens 65 Gew.-%, weiter bevorzugt höchstens 60 Gew.-% der Mantelmischung vorgelegt. Anschließend wird der Kern mit der Zwischenschicht eingebracht und die Form mit dem verbleibenden Anteil an Mantelmischung aufgefüllt. Anschließend wird Pressduck ausgeübt.

Wird der Mantel auf die Zwischenschicht gepresst, so wurde vorzugsweise eine Presskraft von höchstens 35 kN, weiter bevorzugt höchstens 30 kN und besonders bevorzugt höchstens 28 kN angelegt. Werden zu hohe Presskräfte eingesetzt, so werden häufig zu feste Arzneiformen erhalten mit einer damit verbundenen schlechteren Freisetzung des Wirkstoffs. Auch kann es beim Pressen unter zu hohen Pressdrücken, insbesondere bei einer konkaven oder biplanaren Form des Kerns, zur Deformierung des Kerns kommen. Dadurch kann die Zwischenschicht einreißen und das Wirkprinzip kann zerstört werden. Auch kann es zur Fragmentierung des verdichteten Komprimats bei zu hohen Pressdrücken kommen. Die Presskraft sollte aber vorzugsweise 7,5 kN, weiter bevorzugt 8 kN übersteigen. Werden zu niedrige Presskräfte eingesetzt, ist eine unzureichende Festigkeit der Arzneiform zu beobachten.

Die Bruchfestigkeit der Arzneiform sollte vorzugsweise mindestens 60 N, weiter bevorzugt mindestens 100 N und ganz besonders bevorzugt mindestens 120 N betragen. Die Bruchfestigkeit sollte vorzugsweise aber 350 N, bevorzugt 300 N nicht übersteigen. Ganz besonders bevorzugt liegt die Bruchfestigkeit der Arzneiform zwischen 140 N und 280 N, noch mehr bevorzugt zwischen 170 und 270 N. Bei zu hohen Bruchfestigkeiten ist der Zerfall messbar schlechter. Die Bruchfestigkeit einer Tablette kann nach gängigen Verfahren unter Normalbedingungen mit Härtetestern ermittelt werden unter Einwirkung einer diametrisch wirkenden Kraft, in der Regel ein spitzer oder kegelförmiger Prüfkörper. Erfindungsgemäß wurde die Bruchfestigkeit mit einem Erweka TBH-30-Härtemessgerät ermittelt.

Der Durchmesser des Kerns beträgt bevorzugt mindestens 3 mm, weiter bevorzugt mindestens 4 mm. Bei einem zu geringen Durchmesser des Kerns kann die Handhabbarkeit und Verarbeitbarkeit erschwert sein. Der Durchmesser des Kerns beträgt vorzugsweise maximal 16 mm, weiter bevorzugt maximal 15 mm und besonders bevorzugt maximal 14 mm. Ist der Durchmesser des Kerns zu groß, kann die Schluckbarkeit der erhaltenen Arzneiform beeinträchtigt sein. Der Begriff "Durchmesser" bezieht sich auf den Durchmesser des Kerns an seiner jeweils dicksten Stelle.

Die Schichtdicke des Mantels beträgt vorzugsweise mindestens 0,7 mm, weiter bevorzugt mindestens 0,8 mm. Bei zu geringen Schichtdicken des Mantels sind die Handhabbarkeit der erfindungsgemäßen Arzneiform sowie deren Herstellung erschwert. Die Schichtdicke des Mantels beträgt vorzugsweise maximal 5 mm, bevorzugt maximal 3 mm. Bei zu großen Schichtdicken des Mantels kann die Arzneiform zu groß werden und die Schluckbarkeit kann beeinträchtigt sein. Schichtdicke ist dabei die Dicke des Mantels an der jeweils dicksten Stelle.

Der Gesamtdurchmesser der Arzneiform richtet sich nach den jeweils verwendeten Inhaltsstoffen, insbesondere den Wirkstoffen im Kern und Mantel. Ebenso richtet sich der Gesamtdurchmesser der Arzneiform nach dem Durchmesser des Kerns. Der Gesamtdurchmesser der Arzneiform liegt vorzugsweise bei maximal 20 mm, weiter bevorzugt bei maximal 18 mm, ganz besonders bevorzugt bei maximal 16 mm. Bei zu hohen Durchmessern kann die Schluckbarkeit beeinträchtigt sein. Der Begriff "Gesamtdurchmesser" bezieht sich auf den Durchmesser der Arzneiform an ihrer jeweils dicksten Stelle. Als besonders vorteilhaft hat sich ein Durchmesser der Arzneiform zwischen 8 mm und 14 mm, insbesondere zwischen 11 mm und 13 mm erwiesen.

Die Arzneiform hat vorzugsweise eine Masse von höchstens 1100 mg, bevorzugt höchstens 950 mg und am meisten bevorzugt höchstens 850 mg, damit diese leicht eingenommen werden kann. Die Arzneiform weist aber erfindungsgemäß bevorzugt ein Mindestgewicht von 115 mg auf, weiter bevorzugt von 225 mg, damit auch ältere Menschen die Arzneiform gut handhaben können. Besonders vorteilhaft war ein Gewicht der Arzneiform zwischen 700 mg und 800 mg.

Die erfindungsgemäße Arzneiform weist auch bei Lagerung an der Luft vorzugsweise eine Restfeuchte, also einen absoluten Wasseranteil von maximal 15 Gew.-%, bevorzugt maximal 13 Gew.-% auf. Ganz besonders bevorzugt beträgt der absolute Wasseranteil maximal 5 Gew.-%. Dies wird vorzugsweise ermittelt durch Trocknung bei 105°C bis zur Gewichtskonstanz in einem Trockenschrank oder mittels eines Infrarot-Trockners, bevorzugt ist der Infrarot-Trockner.

Die Arzneiformen der Erfindung haben den Vorteil, dass sie besonders lagerstabil sind, dass heißt die Kriterien für Lagerstabilität der ICH sind mindestens erfüllt und vorzugsweise übertroffen, d.h. die erfindungsgemäßen Arzneiformen zeigen bessere Werte als ausreichend. Lagerstabilität bedeutet vorzugsweise, dass während einer Lagerung bei bestimmten Lagerbedingungen und einer bestimmten Lagerdauer, die sich aus der ICH-Leitlinie Q3B (R2) (Impurities in New Drug Products) ergeben, ein ausreichend hoher Arzneistoffgehalt von vorzugsweise mehr als 90%, bezogen auf die ursprüngliche Wirkstoffmenge, zur Verfügung steht, und dass Abbauprodukte, die eine Gefährdung der Patienten darstellen könnten, ein bestimmtes Höchstmaß nicht überschreiten. Die Höchstmaße ergeben sich aus der ICH-Leitlinie Q3B (R2).

Die erfindungsgemäßen Arzneiformen erwiesen sich als äußerst lagerstabil in einer Kurzzeit-Stressuntersuchung in üblichen Blistern (beispielsweise Oberfolie Alu-Folie, 20 µm hart und Unterfolie PVC/PVDC-Folie, glasklar) bei 25°C und 60% relative Luftfeuchte (entspricht subtropischem bis Mittelmeerklima), 30°C und 65% relative Luftfeuchte (entspricht heißem und feuchtem Klima) sowie bei 40°C und 75% relative Luftfeuchte (entspricht sehr heißem und besonders feuchtem Klima). Die erfindungsgemäßen Arzneiformen enthalten auch nach der Lagerung bei 25°C und 60% relative Luftfeuchte, 30°C und 65% relative Luftfeuchte oder bei 40°C und 75% relative Luftfeuchte nach 1 Monat vorzugsweise noch zwischen 95% und 105% der theoretischen Wirkstoffmenge in der Arzneiform, entsprechen also der Spezifikation. Bevorzugt liegt die Wirkstoffmenge auch nach 1 Monat Lagerung unter einer der angegebenen Bedingungen (25°C und 60% relative Luftfeuchte, 30°C und 65% relative Luftfeuchte oder 40°C und 75% relative Luftfeuchte) zwischen 96% und 103% und noch mehr bevorzugt zwischen 97% und 102% bezogen auf die Menge an Wirkstoff in der ungelagerten Arzneiform. Die Gesamtmasse der gelagerten Arzneiform weicht vorzugsweise um weniger als 5%, noch weiter bevorzugt um weniger als 4% und noch mehr bevorzugt um weniger als 2% von der Masse der nicht gelagerten Arzneiform ab bei Lagerung der Arzneiform für 1 Monat bei 25°C und 60% relative Luftfeuchte, 30°C und 65% relative Luftfeuchte oder 40°C und 75% relative Luftfeuchte. Dies zeigt, dass eine Wasseraufnahme aus der Umgebung mit der erfindungsgemäßen Arzneiform üblicherweise umfassend einen hygroskopischen Wirkstoff auch bei extremer Feuchtigkeit der Umgebungsatmosphäre unterbunden werden kann. Auch der Durchmesser der erfindungsgemäßen Arzneiform weicht nach 1 Monat Lagerung in einer der angegebenen Atmosphären vorzugsweise um weniger als 4%, weiter bevorzugt um weniger als 3% und noch mehr bevorzugt um weniger als 2% vom Durchmesser der ungelagerten Arzneiform ab. Die erfindungsgemäßen Arzneiformen sind vorzugsweise lagerstabil über mehr als 6 Monate, bevorzugt wenigstens 12 Monate Lagerdauer unter Standardbedingungen gemäß internationalen Leitlinien der ICH.

Die erfindungsgemäßen Arzneiformen zeichnen sich durch eine exzellente Gleichförmigkeit der Masse und Gleichförmigkeit des Gehalts aus, was durch die Zusammensetzung der Arzneiformen und das Herstellungsverfahren gewährleistet ist. Die Prüfung erfolgt nach den entsprechenden Methoden des Europäischen Arzneibuchs (Ph.Eur. 7). Eine Arzneiform zeigt vorzugsweise eine Gleichförmigkeit der Masse derart, dass die Masse von 20 solcher Arzneiformen vorzugsweise um nicht mehr als 5%, weiter bevorzugt um weniger als 5%, noch weiter bevorzugt um weniger als 4% von der Durchschnittsmasse der Arzneiform abweicht, die sich aus der Masse der 20 Arzneiformen ergibt. Die erfindungsgemäße Arzneiform zeigt bevorzugt eine Gleichförmigkeit des Gehalts derart, dass der Wirkstoffgehalt von 10 solcher Arzneiformen jeweils zwischen 85% und 115%, bevorzugt zwischen 87% und 113% und idealerweise zwischen 95% und 105%, bezogen auf den Durchschnittsgehalt der 10 Arzneiformen an Wirkstoff liegt.

Mit der erfindungsgemäßen Arzneiform und dem erfindungsgemäßen Herstellungsverfahren ist es somit möglich, insbesondere hygroskopische Wirkstoffe, also schwer verarbeitbare Wirkstoffe, so bereitzustellen, dass eine mehrmals tägliche Gabe simuliert wird. Dabei wird der erste Wirkstoffanteil in der ersten Phase sofort freigesetzt. Eine solche Freisetzung ist insbesondere bei einer Kurzzeittherapie vorteilhaft, wenn der gewünschte Plasmaspiegel möglichst schnell erreicht werden soll und gleichzeitig durch nachgelagerte Freisetzungsphasen die Wirkung in die Länge gezogen werden soll. Auch kann ein solches Freisetzungssystem bei einer Langzeittherapie vorteilhaft sein, insbesondere wenn bei langen Dosierungsintervallen gegen Ende des Intervalls die minimale Wirkkonzentration unterschritten wird. Durch die rasche initiale Wirkstofffreisetzung der Folgearzneiform wird der Plasmaspiegel schnell wieder in den erforderlichen Bereich gebracht.

Die Arzneiform hat dabei eine für die orale Einnahme geeignete Größe. Sie zeichnet sich durch eine hohe mechanische Stabilität auch bei langer Lagerung aus. Insbesondere hygroskopische Wirkstoffe, die azide und/oder hoch löslich sind, profitieren von dem erfindungsgemäßen Arzneiformdesign. Damit kann die Tagesdosis solcher Wirkstoffe insbesondere auf eine einmal tägliche Gabe reduziert werden, was sich positiv auf die Patientencompliance und mittelbar auf die Gesundheitskosten auswirken kann.

Die erfindungsgemäße Arzneiform ist insbesondere zur Verabreichung folgender Wirkstoffe und/oder ihrer pharmazeutisch annehmbaren Salze geeignet: Valproinsäure, Carbamazepin, Tetracyclin, Lincomycin, Clindamycin, Erythromycin, Rifampicin, Metformin, Atenolol, Ranitidin, Acetylsalicylsäure, Diclofenac, Omeprazol, Methyldopa, Minoxidil, Betahistin, Dexamethason, Prednisolon, Piracetam, Pravastatin und Gemfibrozil.

Valproinsäure und Carbamazepin dienen insbesondere der Behandlung bei Epilepsie. Tetracyclin, Lincomycin, Clindamycin, Erythromycin und Rifampicin sind zur Behandlung bakterieller Infektionskrankheiten geeignet. Metformin ist bei Diabetes mellitus indiziert. Der Wirkstoff Atenolol wird bei funktionellen Herz-Kreislauf-Beschwerden, Arrhythmien, arterieller Hypertonie und Angina pectoris eingesetzt. Auch Methyldopa und Minoxidil sind zur Behandlung bei Hypertonie geeignet. Die Wirkstoffe Ranitidin und Omeprazol werden insbesondere bei Magen-Darmulcera, Reflux-Ösophagitis und Zollinger-Ellison-Syndrom eingesetzt. Acetylsalicylsäure und Diclofenac sind bei Schmerzen indiziert. Acetylsalicylsäure ist in verminderter Dosis auch zur Thrombozytenaggregationshemmung bei Angina pectoris oder nach akutem Herzinfarkt geeignet.

Der Wirkstoff Betahistin dient der Behandlung des Menière'schen Symptomkomplexes, dessen Symptome Schwindel oft in Verbindung mit Übelkeit und/oder Erbrechen, Tinnitus und Hörverlust beinhalten können. Dexamethason wird als Kortikosteroid bei Autoimmunerkrankungen, Hirnödem und Asthma eingesetzt. Prednisolon als Kortikosteroid insbesondere bei Nebennierenrindeninsuffizienz. Der Wirkstoff Piracetam ist insbesondere bei hirnorganisch bedingten Leistungsstörungen geeignet. Gemfibrozil und Pravastatin werden üblicherweise zur Behandlung bei Hypertriglyceridämie und Hypercholesterinämie eingesetzt.

Erfindungsgemäß ist daher auch die Verwendung der erfindungsgemäßen Arzneiform zur Behandlung eines Patienten bei einer Erkrankung ausgewählt aus Epilepsie, bakterielle Infektionskrankheit, Diabetes mellitus, funktionelle Herz-Kreislauf-Beschwerden, Arrhythmien, Hypertonie, Angina pectoris, Herzinsuffizienz, Magen-Darmulcus, Reflux-Ösophagitis, Zollinger-Ellison-Syndrom, Schmerzen, Schwindel, insbesondere im Zusammenhang mit dem Menière'schen Symptom-Komplex, Autoimmunerkrankung, Hirnödem, Asthma, Nebennierenrindeninsuffizienz, hirnorganisch bedingte Leistungsstörung, Hypertriglyceridämie und Hypercholesterinämie. Bevorzugt wird die Arzneiform dabei einmal täglich eingenommen. Erfindungsgemäß besonders bevorzugt ist die Verwendung der Arzneiform zur Behandlung bei Schwindel im Zusammenhang mit dem Menière'schen Symptom-Komplex.

Erfindungsgemäß ist ebenso ein Verfahren zur Behandlung von einer Erkrankung ausgewählt aus Epilepsie, bakterielle Infektionskrankheit, Diabetes mellitus, funktionelle Herz-Kreislauf-Beschwerden, Arrhythmien, Hypertonie, Angina pectoris, Herzinsuffizienz, Magen-Darmulcus, Reflux-Ösophagitis, Zollinger-Ellison-Syndrom, Schmerzen, Schwindel im Zusammenhang mit dem Menière'schen Symptom-Komplex, Autoimmunerkrankung, Hirnödem, Asthma, hirnorganisch bedingte Leistungsstörung, Hypertriglyceridämie und Hypercholesterinämie, wobei das Verfahren die Verabreichung einer erfindungsgemäßen Arzneiform umfasst. Bevorzugt erfolgt diese Verabreichung einmal täglich.

### Beispiele

**Beispiel 1: Herstellung einer erfindungsgemäßen Arzneiform**

| **Element** | **Inhaltsstoff** | **Menge pro Arzneiform (mg)** | **Funktion** |
|---|---|---|---|
| **Kern** | Betahistindihydrochlorid | 24 | Wirkstoff |
| | Lactose-Monohydrat (Granulac^{®} 230) | 53 | Trägerstoff |
| | Mikrokristalline Cellulose (Vivapur^{®} 102) | 30 | Trägerstoff |
| | Maisstärke | 53 | Trägerstoff |
| | Citronensäure, wasserfrei | 24 | Puffersubstanz |
| | Povidon K25 (Plasdone^{®} K25) | 2 | Granuliermittel |
| | Gehärtetes Baumwollsamenöl (Lubritab^{®}) | 5 | Gleitmittel |
| | Gesamtmasse | 191 | |
| **Zwischenschicht** | Eudragit^{®} S 100 | 6,67 | Filmbildner |
| | Eudragit^{®} L 100 | 1,67 | Filmbildner |
| | Triacetin | 2,50 | Weichmacher |
| | Talkum | 4,17 | Füllstoff |
| | Gesamtmasse | 15,01 | |
| **Mantel** | Betahistindihydrochlorid | 24 | Wirkstoff |
| | Lactose-Monohydrat (Granulac^{®} 230) | 53 | Trägerstoff |
| | Lactose-Monohydrat (Tablettose^{®} 80) | 128,5 | Trägerstoff |
| | Mikrokristalline Cellulose (Vivapur^{®} 102) | 230 | Trägerstoff |
| | Maisstärke | 53 | Trägerstoff |
| | Citronensäure, wasserfrei | 24 | Puffersubstanz |
| | Povidon K25 (Plasdone^{®} K25) | 2 | Granuliermittel |
| | Vorverkleisterte Maisstärke (Starch 1500^{®}) | 30 | Trägerstoff |
| | Gehärtetes Baumwollsamenöl (Lubritab^{®}) | 5,5 | Gleitmittel |
| | Gesamtmasse | 550 | |
| **Arzneiform** | **Masse** | **756,01** | |

Es wurde eine erfindungsgemäße Arzneiform hergestellt in Form einer Mantel-Kern-Tablette. Zunächst wurde der Kern hergestellt. Zur Herstellung des Kerns wurde ein Vorgranulat hergestellt. Dazu wurden die Trägerstoffe Lactose-Monohydrat, Mikrokristalline Cellulose und Maisstärke sowie die Puffersubstanz Citronensäure intensiv gemischt in einem Mischer/Granulierer (Diosna P10). Die Mischung wurde anschließend granuliert in dem Mischer Diosna P10 mit einer Granulierlösung. Die Granulierlösung wurde hergestellt durch Lösen des Povidon K25 in gereinigtem Wasser, so dass die Lösung 16 Gew.-% Povidon K25 enthielt. Das Vorgranulat wurde anschließend in einer Wirbelschichtanlage (GPCG-3) getrocknet bei 60°C auf eine Restfeuchte von < 5%. Das getrocknete Vorgranulat wurde anschließend gesiebt (Siebweite 1 mm).

Aus dem Vorgranulat wurde das Wirkstoffgranulat hergestellt durch Zugabe einer Lösung von Betahistindihydrochlorid in Methanol. Die klare Lösung enthielt dabei 28,5 Gew.-% Betahistindihydrochlorid. Dazu wurde das Vorgranulat vorgelegt in den Mischer/Granulierer Diosna 10 und mit der Wirkstofflösung granuliert. Das erhaltenen Wirkstoffgranulat wurde in einer Wiebelschichtanlage (GPCG-3) getrocknet auf einen Restmethanolgehalt von < 3000 ppm. Das getrocknete Wirkstoffgranulat wurde anschließend gesiebt (Siebweite 1 mm).

Zum Wirkstoffgranulat wurde anschließend gehärtetes Baumwollsamenöl gegeben und in einem Mischer gemischt. Die erhaltene Kernmischung wurde auf einer Rundläuferpresse verpresst. Es wurden bikonvexe Kerne mit einem Durchmesser von 8 mm erhalten. Die erhaltenen Kerne hatten ein Gewicht von 191 mg und eine Höhe von 3,85 mm.

Anschließend wurde die Zwischenschicht hergestellt. Dazu wurden die Filmbildner Eudragit^{®} S 100 und Eudragit^{®} L 100 in *iso*-Propanol gelöst, wobei der Gehalt an Filmbildnern an der Lösung 5,9 Gew.-% betrug. In die Lösung wurden der Weichmacher Triacetin sowie der Hilfsstoff Talkum eingerührt. Ebenso wurde Wasser zugesetzt (6,67 ml). Die Zwischenschichtmischung wurde anschließend auf den Kern aufgetragen, so dass diese den Kern vollständig bedeckte. Das Auftragen der Zwischenschichtmischung erfolgte in einem GS-Coater (GS-10) bei einer Zulufttemperatur von 50°C.

Zur Herstellung des Mantels wurde das Wirkstoffgranulat analog zum Kern hergestellt, so dass 186 mg Wirkstoffgranulat vorlagen. Dieses Wirkstoffgranulat wurde gemischt mit den Trägerstoffen Lactose-Monohydrat, Mikrokristalline Cellulose und vorverkleisterte Maisstärke sowie dem Gleitmittel gehärtetes Baumwollsamenöl. Dies ergibt die Mantelmischung. Der Anteil Wirkstoffgranulat an der Mantelmischung betrug somit 33,8 Gew.-%.

Die Mantelmischung wurde im letzten Schritt auf die Zwischenschicht aufgepresst in einer Syl-one Presse mit Tablettenzuführung. Dazu wurden 300 mg der Mantelmischung in eine Matrize (rund, bikonvex, Durchmesser 12 mm, Wölbungsradius 9,5 mm) eingefüllt. Darauf wurde der Kern mit der Zwischenschicht zentrisch positioniert. In einem zweiten Füllschritt wurde der verbleibende Anteil der Mantelmischung in die Matrize gefüllt. Anschließend wurde mit einer Presskraft von 10 kN gepresst. Die Mantel/Kern-Tablette hatte eine Bruchfestigkeit von 140 N, gemessen mit einem Erweka TBH-30-Härtemessgerät. In einer weiteren Ausführung wurde bei gleichen Ausgangsstoffen, Mengenverhältnissen und Herstellungsverfahren eine Presskraft von 25 kN bei dem Pressen des Mantels angelegt, so dass eine Mantel/Kern-Tablette mit einer Bruchfestigkeit von 280 N erhalten wurde.

**Beispiel 2: Herstellung einer erfindungsgemäßen Arzneiform**

| **Element** | **Inhaltsstoff** | **Menge pro Arzneiform (mg)** | **Funktion** |
|---|---|---|---|
| **Zwischenschicht** | Eudragit^{®} S 100 | 8,874 | Filmbildner |
| | Eudragit^{®} L 100 | 2,225 | Filmbildner |
| | Triacetin | 3,331 | Weichmacher |
| | Talkum | 5,556 | Füllstoff |
| | **Gesamtmasse** | **19,986** | |

| | | | |
|---|---|---|---|
| Es wurde eine Mantel-Kern-Tablette hergestellt mit der Zusammensetzung des Kerns und des Mantels wie in Beispiel 1. | | | |

Allerdings wurde die Zusammensetzung der Zwischenschicht geändert, wobei ein höheres Gewicht der Zwischenschicht gewählt wurde. Das gewählte höhere Gewicht an Zwischenschicht trug zu einer weiteren Erhöhung der mechanischen Festigkeit des Kerns bei. Die gewählte Zwischenschicht löst sich bei einem pH-Wert von 7,0 bis 7,2 auf. Die Zwischenschicht ermöglicht dadurch die Freisetzung des Wirkstoffanteils aus dem Kern in den mittleren Darmabschnitt.

Die Herstellung von Kern und Mantel erfolgte wie in Beispiel 1 beschrieben. Die Herstellung der Zwischenschicht erfolgte durch Lösen der Filmbildner Eudragit^{®} S 100 und Eudragit^{®} L 100 in *iso*-Propanol, wobei der Gehalt an Filmbildnern an der Lösung 5,9 Gew.-% betrug. In die Lösung wurden der Weichmacher Triacetin sowie der Hilfsstoff Talkum eingerührt. Ebenso wurde Wasser zugesetzt (8,86 ml). Die Zwischenschichtmischung wurde anschließend auf den Kern aufgetragen, so dass diese den Kern vollständig bedeckte. Das Auftragen der Zwischenschichtmischung erfolgte in einem GS-Coater (GS-10) bei einer Zulufttemperatur von 50°C.

**Beispiel 3: Herstellung einer erfindungsgemäßen Arzneiform**

| **Element** | **Inhaltsstoff** | **Menge pro Arzneiform (mg)** | **Funktion** |
|---|---|---|---|
| **Zwischenschicht** | Eudragit^{®} S 100 | 11,099 | Filmbildner |
| | Triacetin | 3,331 | Weichmacher |
| | Talkum | 5,556 | Füllstoff |
| | **Gesamtmasse** | **19,986** | |

| | | | |
|---|---|---|---|
| Es wurde eine Mantel-Kern-Tablette hergestellt mit der Zusammensetzung des Kerns und des Mantels wie in Beispiel 1. Allerdings wurde die Zusammensetzung der Zwischenschicht geändert, wobei ein höheres Gewicht der | | | |

Zwischenschicht gewählt wurde analog zum Beispiel 2. Das gewählte höhere Gewicht an Zwischenschicht trug zu einer weiteren Erhöhung der mechanischen Festigkeit des Kerns bei. Die gewählte Zwischenschicht löst sich bei einem pH-Wert von 7,2 bis 7,5 auf. Die Zwischenschicht ermöglicht dadurch die Freisetzung des Wirkstoffanteils aus dem Kern in den unteren Darmabschnitt.

Die Herstellung von Kern und Mantel erfolgte wie in Beispiel 1 beschrieben. Die Herstellung der Zwischensicht erfolgte durch Lösen des Filmbildners Eudragit^{®} S 100 in *iso*-Propanol, wobei der Gehalt an Filmbildner an der Lösung 5,9 Gew.-% betrug. In die Lösung wurden der Weichmacher Triacetin sowie der Hilfsstoff Talkum eingerührt. Ebenso wurde Wasser zugesetzt (8,86 ml). Die Zwischenschichtmischung wurde anschließend auf den Kern aufgetragen, so dass diese den Kern vollständig bedeckte. Das Auftragen der Zwischenschichtmischung erfolgte in einem GS-Coater (GS-10) bei einer Zulufttemperatur von 50°C.

### Beispiel 4:

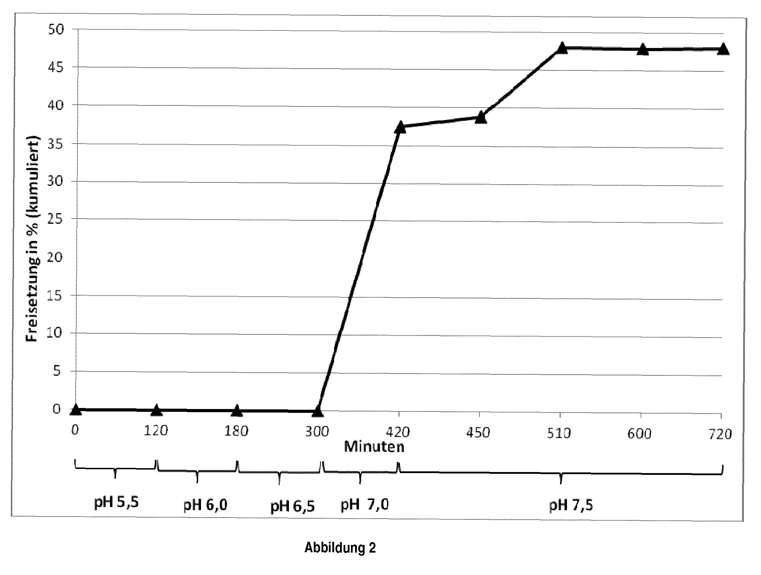

Es wurde im Beispiel 4 ein Versuch unternommen zu Freisetzung des Wirkstoffs Betahistindihydrochlorid aus einem Kern mit Zwischenschicht mit der Zusammensetzung gemäß Beispiel 1, jedoch ohne Mantel. Der Kern mit Zwischenschicht umfasst also 24 mg Betahistindihydrochlorid. Die Freisetzung wurde mit der Paddle-Apparatur ermittelt. Abbildung 2 zeigt die entsprechenden Ergebnisse.

### Beispiel 5: Stabilität der erfindungsgemäßen Arzneiform

Es wurde eine erfindungsgemäße Arzneiform hergestellt in Form einer Mantel-Kern-Tablette mit der Zusammensetzung des Kerns und des Mantels wie in Beispiel 1. Blister (Oberfolie Alu-Folie 20 µm hart, Unterfolie PVC/PVDC-Folie, glasklar) umfassend die Arzneiform wurden für 1 Monat einem Stresstest unterzogen, also bei 25°C/60% relative Luftfeuchte, 30°C/65% relative Luftfeuchte und 40°C/75% relative Luftfeuchte gelagert. Die Arzneiform zeige nach der Lagerung die nachfolgend aufgeführten Parameter. Es wird deutlich, dass die erfindungsgemäße Arzneiform auch unter extremen Lagerbedingungen stabil ist und trotz des hygroskopischen Wirkstoffs nicht deckelt bzw. quillt. Die Verunreinigungen gemäß Spezifikation bzw. Monographie nach Ph. Eur. 7 lagen auch nach 1 Monat Lagerung bei den extremen Bedingungen unter den entsprechend definierten Grenzwerten bzw. waren nicht in messbarer Menge vorhanden.

| **Parameter** | **ungelagerte Arzneiform** | **Lagerung 1 Monat bei 25°C/60% relative Luftfeuchte** | **Lagerung 1 Monat bei 30°C/65% relative Luftfeuchte** | **Lagerung 1 Monat bei 40°C/75% relative Luftfeuchte** |
|---|---|---|---|---|
| Höhe (mm) | 7,1 | 7,1 | 7,1 | 7,2 |
| Durchmesser (mm) | 12,0 | 12,0 | 12,0 | 12,1 |
| tatsächliches Gewicht (mg) | 755,7 | 757,6 | 757,4 | 767,0 |
| Gehalt (% der theoretischen Wirkstoffmenge) | 100,6 | 101,1 | 101,5 | 100,8 |

### Beschreibung der Abbildungen:

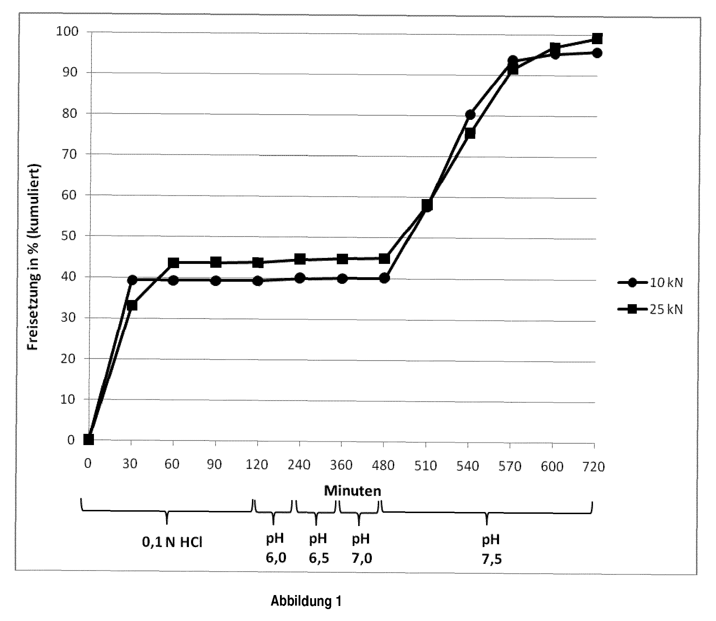

Abbildung 1 zeigt den Freisetzungsverlauf von zwei erfindungsgemäßen Arzneiformen hergestellt gemäß dem Ausführungsbeispiel 1 umfassend 24 mg Betahistindihydrochlorid im Mantel und 24 mg Betahistindihydrochlorid im Kern. Der Mantel wurde mit einer Presskraft von 10 kN oder 25 kN auf den Kern mit Zwischenschicht gepresst. Dabei ist ein den physiologischen Bedingungen entsprechender pH-Verlauf nachgeahmt. Eine 100%-ige Freisetzung bedeutet eine Freisetzung von 48 mg Betahistindihydrochlorid aus der Arzneiform, also 24 mg aus dem Mantel und 24 mg aus dem Kern. Die erfindungsgemäße Arzneiform setzt Betahistindihydrochlorid zweiphasig frei, also gepulst zunächst aus dem Mantel und ab einem pH 7,0 (nach etwa 8,5 Stunden) nach Auflösung der magensaftresistenten Zwischenschicht aus dem Kern. Es wird also eine zweimal tägliche Gabe simuliert, also die Einnahme zweier kommerziell erhältlicher Arzneiformen ohne besondere Freisetzungsmodifikation im Abstand von 8 bis 12 Stunden. Durch die besondere Ausgestaltung der Arzneiform wird ein optimales Freisetzungsprofil erzielt. Es ist zu erwarten, dass sich dies auch *in-vivo* bestätigen wird.

Abbildung 2 zeigt den Freisetzungsverlauf aus dem Kern mit Zwischenschicht gemäß Beispiel 4. Eine Freisetzung von 50% entspricht der Freisetzung von 24 mg Betahistindihydrochlorid. Es ist erkennbar, dass erst ab einem pH-Wert von 7,0 die Auflösung der Zwischenschicht erfolgt und der Wirkstoffanteil aus dem Kern freigesetzt wird. Der Kern mit Zwischenschicht ist also in oberen Darmabschnitten stabil, der Zerfall setzt erst ab einem pH-Wert ab 7,0 ein, was dem Darmabschnitt Ileum zu Colon und einer Verweilzeit im Gastrointestinaltrakt ohne Freisetzung von 7 bis 12 Stunden entspricht.

## Patentansprüche

1. Arzneiform umfassend
a. einen Kern,
b. eine magensaftresistente Zwischenschicht, die auf der Oberfläche des Kerns angeordnet ist, und
c. einen Mantel, der auf der dem Kern gegenüberliegenden Seite der Zwischenschicht angeordnet ist,
wobei sowohl der Kern als auch der Mantel jeweils einen Anteil eines Wirkstoffs enthalten, und wobei die Arzneiform den Wirkstoff mindestens zweiphasig freisetzt, wobei die erste Phase die sofortige Freisetzung des Wirkstoffs nach Einnahme in den Magensaft betrifft und wobei das Masseverhältnis von Mantel zu Kern einen Wert von wenigstens 0,5:1 hat.

2. Arzneiform nach Anspruch 1, wobei die Zwischenschicht einen Filmbildner umfasst.

3. Arzneiform nach Anspruch 1 oder 2, wobei insgesamt zwischen 1 mg und 200 mg des Wirkstoffes in der Arzneiform enthalten sind.

4. Arzneiform nach wenigstens einem der vorhergehenden Ansprüche, wobei der Kern wenigstens einen Trägerstoff aufweist.

5. Arzneiform nach wenigstens einem der vorhergehenden Ansprüche, wobei der Kern wenigstens eine Puffersubstanz umfasst.

6. Arzneiform nach wenigstens einem der vorhergehenden Ansprüche, wobei der Anteil des Wirkstoffs im Kern und im Mantel jeweils 40% bis 60% der Gesamtmenge des Wirkstoffes in der Arzneiform beträgt.

7. Arzneiform nach wenigstens einem der vorhergehenden Ansprüche, wobei das Masseverhältnis von Mantel zu Kern einen Wert von wenigstens 1,8:1 hat.

8. Arzneiform nach wenigstens einem der vorhergehenden Ansprüche, wobei das Masseverhältnis von Kern zu Zwischenschicht einen Wert von mindestens 3:1 hat.

9. Arzneiform nach wenigstens einem der vorhergehenden Ansprüche, wobei das Masseverhältnis von Kern zu Zwischenschicht höchstens einen Wert von 40:1 hat.

10. Arzneiform nach wenigstens einem der vorhergehenden Ansprüche, wobei der Wirkstoff Betahistin oder ein pharmazeutisch geeignetes Salz von Betahistin ist.

11. Arzneiform nach wenigstens einem der vorhergehenden Ansprüche, wobei der Wirkstoff ausgewählt ist aus Betahistindihydrochlorid und Betahistindimesilat, und wobei zwischen 2 mg und 55 mg bezogen auf die Wirkstoffbase in der Arzneiform enthalten sind.

12. Arzneiform nach wenigstens einem der vorhergehenden Ansprüche, wobei die Arzneiform aus dem Kern, der Zwischenschicht und dem Mantel besteht.

13. Arzneiform nach wenigstens einem der vorhergehenden Ansprüche, wobei die Arzneiform eine Mantel-Kern-Tablette ist.

14. Verfahren zur Herstellung einer Arzneiform nach wenigstens einem der Ansprüche 1 bis 13 mit den Schritten
a. Herstellen des Kerns,
b. Herstellen der Zwischenschicht,
c. Herstellen des Mantels.

15. Arzneiform nach einem der Ansprüche 1 bis 13 zur Verwendung in einem Therapieverfahren.

## Claims

1. A medicament form, comprising
a. a core,
b. a gastric juice-resistant intermediate layer being disposed on the surface of the core, and
c. a shell being disposed on the opposite side of the intermediate layer from the core, wherein both the core and the shell contain a proportion of an active ingredient each, and wherein the medicament form releases the active ingredient in an at least biphasic form, wherein the first phase relates to the immediate release of the active ingredient after ingestion into the gastric juice, wherein the mass ratio of shell to core has a value of at least 0.5:1.

2. The medicament form according to claim 1, wherein the intermediate layer comprises a film-forming component.

3. The medicament form according to claim 1 or 2, wherein in total between 1 mg and 200 mg of the active ingredient are contained in the medicament form.

4. The medicament form according to at least one of the preceding claims, wherein the core comprises at least one carrier.

5. The medicament form according to at least one of the preceding claims, wherein the core comprises at least one buffer substance.

6. The medicament form according to at least one of the preceding claims, wherein the proportion of the active ingredient in the core and in the shell each amounts to 40 % to 60 % of the total amount of the active ingredient in the medicament form.

7. The medicament form according to at least one of the preceding claims, wherein the mass ratio of shell to core has a value of at least 1.8:1.

8. The medicament form according to at least one of the preceding claims, wherein the mass ratio of core to intermediate layer has a value of at least 3:1.

9. The medicament form according to at least one of the preceding claims, wherein the mass ratio of core to intermediate layer has a value of at most 40:1.

10. The medicament form according to at least one of the preceding claims, wherein the active ingredient is betahistine or a pharmaceutically acceptable salt of betahistine.

11. The medicament form according to at least one of the preceding claims, wherein the active ingredient is selected from betahistine dihydrochloride and betahistine dimesylate, and wherein between 2 mg and 55 mg, based on the base of the active ingredient, are contained in the medicament form.

12. The medicament form according to at least one of the preceding claims, wherein the medicament form consists of the core, the intermediate layer and the shell.

13. The medicament form according to at least one of the preceding claims, wherein the medicament form is a shell-core tablet.

14. A method for the production of a medicament form according to at least one of claims 1 to 13 with the steps
a. preparing the core,
b. preparing the intermediate layer,
c. preparing the shell.

15. The medicament form according to one of claims 1 to 13 for use in a therapy method.

## Revendications

1. Forme pharmaceutique comprenant
a. un noyau,
b. une couche intermédiaire résistante aux sucs gastriques qui est disposée sur la surface du noyau, et
c. un revêtement qui est disposé sur le côté opposé au noyau de la couche intermédiaire,
dans laquelle aussi bien le noyau que le revêtement contiennent respectivement une proportion d'un principe actif, et dans laquelle la forme pharmaceutique libère le principe actif au moins en deux phases, dans laquelle la première phase concerne la libération immédiate du principe actif après absorption dans le suc gastrique et dans laquelle le rapport en masse du revêtement au noyau a une valeur d'au moins 0,5:1.

2. Forme pharmaceutique selon la revendication 1, dans laquelle la couche intermédiaire comprend un agent filmogène.

3. Forme pharmaceutique selon la revendication 1 ou 2, dans laquelle au total, entre 1 mg et 200 mg du principe actif sont contenus dans la forme pharmaceutique.

4. Forme pharmaceutique selon au moins l'une des revendications précédentes, dans laquelle le noyau présente au moins un véhicule.

5. Forme pharmaceutique selon au moins l'une des revendications précédentes, dans laquelle le noyau comprend au moins une substance tampon.

6. Forme pharmaceutique selon au moins l'une des revendications précédentes, dans laquelle la proportion du principe actif dans le noyau et dans le revêtement est respectivement de 40 % à 60 % de la quantité totale du principe actif dans la forme pharmaceutique.

7. Forme pharmaceutique selon au moins l'une des revendications précédentes, dans laquelle le rapport en masse du revêtement au noyau a une valeur d'au moins 1,8:1.

8. Forme pharmaceutique selon au moins l'une des revendications précédentes, dans laquelle le rapport en masse du noyau à la couche intermédiaire est d'au moins 3:1.

9. Forme pharmaceutique selon au moins l'une des revendications précédentes, dans laquelle le rapport en masse du noyau à la couche intermédiaire a une valeur maximale de 40:1.

10. Forme pharmaceutique selon au moins l'une des revendications précédentes, dans laquelle le principe actif est la bêtahistine ou un sel pharmaceutiquement approprié de la bêtahistine.

11. Forme pharmaceutique selon au moins l'une des revendications précédentes, dans laquelle le principe actif est choisi parmi le dichlorhydrate de bêtahistine et le dimésilate de bêtahistine, et dans laquelle entre 2 mg et 55 mg par rapport à la base de principe actif sont contenus dans la forme pharmaceutique.

12. Forme pharmaceutique selon au moins l'une des revendications précédentes, dans laquelle la forme pharmaceutique est constituée du noyau, de la couche intermédiaire et du revêtement.

13. Forme pharmaceutique selon au moins l'une des revendications précédentes, dans laquelle la forme pharmaceutique est un comprimé à revêtement-noyau.

14. Procédé de fabrication d'une forme pharmaceutique selon au moins l'une des revendications 1 à 13, comportant les étapes de
a. fabrication du noyau,
b. fabrication de la couche intermédiaire,
c. fabrication du revêtement.

15. Forme pharmaceutique selon l'une des revendications 1 à 13 pour l'utilisation dans un processus thérapeutique.
